(19) Europäisches Patentamt / European Patent Office / Office européen des brevets

(11) **EP 4 189 037 B1**

(12) **FASCICULE DE BREVET EUROPÉEN**

(45) Date de publication et mention
de la délivrance du brevet:
**18.09.2024 Bulletin 2024/38**

(21) Numéro de dépôt: **21751786.1**

(22) Date de dépôt: **26.07.2021**

(51) Classification Internationale des Brevets (IPC):
*C10G 1/10* (2006.01)     *C10G 9/36* (2006.01)
*C10G 67/04* (2006.01)

(52) Classification Coopérative des Brevets (CPC):
**C10G 65/12; C10G 1/002; C10G 1/10; C10G 9/36;
C10G 67/04; C10G 67/0418; C10G 67/0427;**
C10G 2400/02

(86) Numéro de dépôt international:
**PCT/EP2021/070849**

(87) Numéro de publication internationale:
**WO 2022/023262 (03.02.2022 Gazette 2022/05)**

(54) **PROCÉDÉ DE TRAITEMENT D'HUILES DE PYROLYSE DE PLASTIQUES INCLUANT UN HYDROCRAQUAGE EN UNE ÉTAPE**

HYDROCRACKINGSCHRITTVERFAHREN ZUR BEHANDLUNG VON KUNSTSTOFFPYROLYSEÖLEN MIT EINEM HYDROCRACKINGSCHRITT

PROCESS FOR TREATING PLASTIC PYROLYSIS OILS INCLUDING ONE-STEP HYDROCRACKING

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **30.07.2020 FR 2008106**

(43) Date de publication de la demande:
**07.06.2023 Bulletin 2023/23**

(73) Titulaires:
• **IFP Energies nouvelles
92500 Rueil-Malmaison (FR)**
• **Repsol, S.A.
28045 Madrid (ES)**

(72) Inventeurs:
• **WEISS, Wilfried
92852 RUEIL-MALMAISON CEDEX (FR)**
• **BONNARDOT, Jérôme
92500 RUEIL-MALMAISON (FR)**
• **RIBAS SANGÜESA, Iñigo
28935 MOSTOLES (ES)**

(74) Mandataire: **IFP Energies nouvelles
Département Propriété Industrielle
Rond Point de l'échangeur de Solaize
BP3
69360 Solaize (FR)**

(56) Documents cités:
**WO-A1-2014/001632     US-A- 3 492 220
US-A- 5 969 201**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

**DOMAINE TECHNIQUE**

La présente invention concerne un procédé de traitement d'une huile de pyrolyse de plastiques afin d'obtenir un effluent hydrocarboné qui peut être valorisé par exemple en étant au moins en partie directement intégré à un pool naphta ou diesel ou comme charge d'une unité de vapocraquage. Plus particulièrement, la présente invention concerne un procédé de traitement d'une charge issue de la pyrolyse des déchets plastiques, afin d'éliminer au moins en partie des impuretés, notamment les oléfines (mono-, di- oléfines), les métaux, en particulier le silicium, et les halogènes, en particulier le chlore, que ladite charge peut contenir en quantités relativement importantes, et de manière à hydrogéner la charge pour pouvoir la valoriser.

Le procédé selon l'invention permet donc de traiter les huiles de pyrolyse de plastiques pour obtenir un effluent qui peut être injecté, en tout ou partie, dans une unité de vapocraquage. Le procédé selon l'invention permet ainsi de valoriser les huiles de pyrolyse de plastiques, tout en en réduisant la formation de coke et ainsi les risques de bouchage et/ou de pertes prématurées d'activité du/des catalyseurs utilisés dans l'unité de vapocraquage, et en diminuant les risques de corrosion.

**TECHNIQUE ANTERIEURE**

Les plastiques issus des filières de collecte et de tri peuvent subir une étape de pyrolyse afin d'obtenir entre autres des huiles de pyrolyse. Ces huiles de pyrolyse de plastiques sont généralement brûlées pour générer de l'électricité et/ou utilisées en tant que combustible dans des chaudières industrielles ou de chauffage urbain.

Une autre voie de valorisation des huiles de pyrolyse de plastiques est l'utilisation de ces huiles de pyrolyse de plastiques en tant que charge d'une unité de vapocraquage afin de (re)créer des oléfines, ces dernières étant des monomères constitutifs de certains polymères. Cependant, les déchets plastiques sont généralement des mélanges de plusieurs polymères, par exemple des mélanges de polyéthylène, de polypropylène, de polyéthylène téréphtalate, de polychlorure de vinyle, de polystyrène. De plus, en fonction des usages, les plastiques peuvent contenir, en plus des polymères, d'autres composés, comme des plastifiants, des pigments, des colorants ou encore des résidus de catalyseurs de polymérisation. Les déchets plastiques peuvent en outre contenir, de manière minoritaire, de la biomasse provenant par exemple des ordures ménagères. Il en résulte que les huiles issues de la pyrolyse des déchets plastiques comprennent beaucoup d'impuretés, en particulier des dioléfines, des métaux, notamment le silicium, ou encore des composés halogénés, notamment des composés à base de chlore, des hétéroéléments comme du soufre, de l'oxygène et de l'azote, des insolubles, à des teneurs souvent élevées et incompatibles avec les unités de vapocraquage ou les unités situées en aval des unités de vapocraquage, notamment les procédés de polymérisation et les procédés d'hydrogénation sélective. Ces impuretés peuvent générer des problèmes d'opérabilité et notamment des problèmes de corrosion, de cokage ou de désactivation catalytique, ou encore des problèmes d'incompatibilité dans les usages des polymères cibles. La présence de dioléfines peut également conduire à des problèmes d'instabilité de l'huile de pyrolyse se caractérisant par la formation de gommes. Les gommes et les insolubles éventuellement présents dans l'huile de pyrolyse peuvent générer des problèmes de colmatage dans les procédés.

De plus, lors de l'étape de vapocraquage, les rendements en oléfines légères recherchées pour la pétrochimie, notamment l'éthylène et le propylène, dépendent fortement de la qualité des charges envoyées au vapocraquage. Le BMCI (Bureau of Mines Corrélation Index selon la terminologie anglo-saxonne) est souvent utilisé pour caractériser les coupes hydrocarbonées. Globalement, les rendements en oléfines légères augmentent quand la teneur en paraffines augmente et/ou quand le BMCI diminue. A l'inverse, les rendements en composés lourds non recherchés et/ou en coke augmentent quand le BMCI augmente.

Le document WO 2018/055555 propose un procédé de recyclage des déchets plastiques global, très général et relativement complexe, allant de l'étape même de pyrolyse des déchets plastiques jusqu'à l'étape de vapocraquage. Le procédé de la demande WO 2018/055555 comprend, entre autres, une étape d'hydrotraitement de la phase liquide issue directement de la pyrolyse, de préférence dans des conditions assez poussées notamment en termes de température, par exemple à une température comprise entre 260 et 300°C, une étape de séparation de l'effluent d'hydrotraitement puis une étape d'hydrodealkylation de l'effluent lourd séparé à une température de préférence élevée, par exemple comprise entre 260 et 400°C.

La demande de brevet non publiée FR20/01.758 décrit un procédé de traitement d'une huile de pyrolyse de plastiques, comprenant :

a) l'hydrogénation sélective de ladite charge en présence d'hydrogène et d'un catalyseur d'hydrogénation sélective pour obtenir un effluent hydrogéné ;

b) l'hydrotraitement dudit effluent hydrogéné en présence d'hydrogène et d'un catalyseur d'hydrotraitement, pour obtenir un effluent d'hydrotraitement ;

c) une séparation de l'effluent d'hydrotraitement en présence d'un flux aqueux, à une température entre 50 et 370°C, pour obtenir un effluent gazeux, un effluent liquide aqueux et un effluent liquide hydrocarboné ;

d) optionnellement une étape de fractionnement de tout ou partie de l'effluent hydrocarboné issu de l'étape c), pour obtenir un flux gazeux et au moins deux flux hydrocarbonés qui peuvent être une coupe naphta et une coupe plus lourde ;

e) une étape de recyclage comprenant une phase de récupération d'une fraction de l'effluent hydrocarboné issu de l'étape c) de séparation ou une fraction du et/ou d'au moins un des flux hydrocarboné(s) issu(s) de l'étape d) de fractionnement, vers l'étape a) d'hydrogénation sélective et/ou l'étape b) d'hydrotraitement.

Selon la demande FR20/01.758, la coupe naphta issue de l'étape de fractionnement peut être envoyée, en tout ou partie, soit vers une unité de vapocraquage, soit vers un pool naphta issu de charges pétrolières conventionnelles, soit être recyclée selon l'étape e).

La coupe plus lourde issue de l'étape de fractionnement peut être envoyée, en tout ou partie, soit vers une unité de vapocraquage, soit vers un pool diesel ou kérosène issu de charges pétrolières conventionnelles, soit être recyclée selon l'étape e).

Bien que la coupe plus lourde puisse être envoyée vers une unité de vapocraquage, peu de raffineurs favorisent cette option. En effet, la coupe plus lourde a un BMCI élevé et contient par rapport à la coupe naphta plus de composés naphténiques, naphténo-aromatiques et aromatiques menant ainsi à un ratio C/H plus élevé. Ce ratio élevé est une cause de cokage dans le vapocraqueur, nécessitant ainsi des fours de vapocraquage dédiés à cette coupe. De plus, le vapocraquage d'une telle coupe lourde produit moins de produits d'intérêts qui sont notamment l'éthylène et le propylène mais davantage d'essence de pyrolyse.

Il serait donc avantageux de minimiser le rendement de la coupe lourde et de maximiser le rendement de la coupe naphta en transformant la coupe lourde au moins en partie en coupe naphta par hydrocraquage. Ceci permet d'obtenir plus de naphta qui est de préférence envoyée en vapocraquage pour produire plus d'oléfines tout en réduisant en particulier les risques de bouchage lors d'étapes de traitement d'huiles de pyrolyse des plastiques, comme celles décrites dans l'art antérieur, et la formation de coke en quantités importantes et/ou les risques de corrosion rencontrés lors d'étape(s) ultérieure(s), par exemple lors d'étape de vapocraquage des huiles de pyrolyse des plastiques. La coupe lourde qui n'a pas été transformée par hydrocraquage quant à elle est de préférence valorisée en la recyclant dans l'étape d'hydrocraquage afin d'être recraquée. De plus, les composés C2 à C4 produits lors de l'hydrocraquage peuvent également être envoyés dans le vapocraquage ce qui permet d'améliorer les rendements en oléfines légères (éthylène et propylène). Au global, le rendement en oléfines est au moins maintenu, voire amélioré, tout en éliminant la nécessité d'un four de vapocraquage dédié à la coupe lourde.

WO 2014/001632 divulgue un procédé de traitement de biomasse pouvant contenir des polymères comprenant une préhydrogénation en deux étapes, une étape d'hydroprocessing et une étape de séparation. US 3 492 220 divulgue un procédé de traitement d'une charge fossile comprenant une étape d'hydrogénation sélective, une étape d'hydrotraitement et une étape de séparation. US 5 969 201 divulgue un procédé de traitement de plastiques comprenant des composés chlorés comprenant une étape d'hydrotraitement, une étape d'hydrocraquage, une étape de séparation de l'effluent en une fraction gazeuse et une fraction liquide et une étape de séparation de la fraction gazeuse alimentée par une solution aqueuse,

## RESUME DE L'INVENTION

L'invention concerne un procédé de traitement d'une charge comprenant une huile de pyrolyse de plastiques comprenant des composés chlorés, comprenant :

a) une étape d'hydrogénation sélective mise en oeuvre dans une section réactionnelle alimentée au moins par ladite charge et un flux gazeux comprenant de l'hydrogène, en présence d'au moins un catalyseur d'hydrogénation sélective, à une température entre 100 et 280°C, une pression partielle d'hydrogène entre 1,0 et 10,0 MPa abs. et une vitesse volumique horaire entre 0,3 et 10,0 $h^{-1}$, pour obtenir un effluent hydrogéné ;

b) une étape d'hydrotraitement mise en oeuvre dans une section réactionnelle d'hydrotraitement, mettant en oeuvre au moins un réacteur à lit fixe ayant n lits catalytiques, n étant un nombre entier supérieur ou égal à 1, comprenant chacun au moins un catalyseur d'hydrotraitement, ladite section réactionnelle d'hydrotraitement étant alimentée au moins par ledit effluent hydrogéné issu de l'étape a) et un flux gazeux comprenant de l'hydrogène, ladite section réactionnelle d'hydrotraitement étant mise en oeuvre à une température entre 250 et 430°C, une pression partielle d'hydrogène entre 1,0 et 10,0 MPa abs. et une vitesse volumique horaire entre 0,1 et 10,0 h$^{-1}$, pour obtenir un effluent d'hydrotraitement ;

c) une étape d'hydrocraquage mise en oeuvre dans une section réactionnelle d'hydrocraquage, mettant en oeuvre au moins un réacteur à lit fixe ayant n lits catalytiques, n étant un nombre entier supérieur ou égal à 1, comprenant chacun au moins un catalyseur d'hydrocraquage, ladite section réactionnelle d'hydrocraquage étant alimentée au moins par ledit effluent hydrotraité issu de l'étape b) et un flux gazeux comprenant de l'hydrogène, ladite section réactionnelle d'hydrocraquage étant mise en oeuvre à une température entre 250 et 480°C, une pression partielle d'hydrogène entre 1,5 et 25,0 MPa abs. et une vitesse volumique horaire entre 0,1 et 10,0 h$^{-1}$, pour obtenir un effluent hydrocraqué ;

d) une étape de séparation, alimentée par l'effluent hydrocraqué issu de l'étape c) et une solution aqueuse, ladite étape étant opérée à une température entre 50 et 370°C, pour obtenir au moins un effluent gazeux, un effluent aqueux et un effluent hydrocarboné.

Un avantage du procédé selon l'invention est de purifier une huile issue de la pyrolyse de déchets plastiques d'au moins une partie de ses impuretés ce qui permet de l'hydrogéner et ainsi de pouvoir la valoriser en particulier en l'incorporant directement à un pool carburant ou encore en la rendant compatible à un traitement dans une unité de vapocraquage afin de pouvoir obtenir en particulier des oléfines légères avec des rendements accrus qui pourront servir de monomères dans la fabrication de polymères.

Un autre avantage de l'invention est de prévenir des risques de bouchage et/ou de corrosion de l'unité de traitement dans laquelle le procédé de l'invention est mis en oeuvre, les risques étant exacerbés par la présence, souvent en quantités importantes, de dioléfines, de métaux et de composés halogénés dans l'huile de pyrolyse de plastiques.

Le procédé de l'invention permet ainsi d'obtenir un effluent hydrocarboné issu d'une huile de pyrolyse de plastiques débarrassé au moins en partie des impuretés de l'huile de pyrolyse de plastiques de départ, limitant ainsi les problèmes d'opérabilité, comme les problèmes de corrosion, de cokage ou de désactivation catalytique, que peuvent engendrer ces impuretés, en particulier dans les unités vapocraquage et/ou dans les unités situées en aval des unités de vapocraquage, notamment les unités de polymérisation et d'hydrogénation sélective. L'élimination d'au moins une partie des impuretés des huiles issues de la pyrolyse des déchets plastiques permettra aussi d'augmenter la gamme des applications des polymères cibles, les incompatibilités d'usages étant réduites.

La présente invention participe au recyclage des plastiques, en proposant un procédé de traitement d'une huile issue de la pyrolyse de plastiques pour la purifier, l'hydrotraiter et l'hydrocraquer afin d'obtenir un effluent hydrocarboné à teneur réduite en impuretés et donc valorisable, soit directement sous forme de coupe naphta et/ou de coupe diesel, soit présentant une composition compatible avec une charge d'une unité de vapocraquage. Le fait d'hydrocraquer permet de transformer au moins une partie de la coupe lourde (diesel) en composés de la coupe naphta ce qui permet d'obtenir des rendements améliorés en coupe naphta et, lorsque cette coupe est envoyée en vapocraquage, en oléfines légères, tout en réduisant en particulier les risques de bouchage lors d'étapes de traitement d'huiles de pyrolyse des plastiques, comme celles décrites dans l'art antérieur, et la formation de coke en quantités importantes et/ou les risques de corrosion rencontrés lors d'étape(s) ultérieure(s), par exemple lors d'étape de vapocraquage des huiles de pyrolyse des plastiques.

Selon une variante, le procédé comprend en outre une étape e) fractionnement de tout ou partie de l'effluent hydrocarboné issu de l'étape d), pour obtenir au moins un flux gazeux et au moins deux flux hydrocarbonés liquides, lesdits deux flux hydrocarbonés liquides étant au moins une coupe naphta comprenant des composés ayant un point d'ébullition inférieur ou égal à 175°C et une coupe hydrocarbonée comprenant des composés ayant un point d'ébullition supérieur à 175°C.

Selon une variante, le procédé comprend en outre une étape f) de recyclage dans laquelle au moins une fraction de la coupe comprenant des composés ayant un point d'ébullition supérieur à 175°C issue de l'étape e) de fractionnement est envoyée vers l'étape c) d'hydrocraquage.

Selon une variante, le procédé comprend en outre une étape g) de recyclage dans laquelle une fraction de l'effluent hydrocarboné issu de l'étape d) de séparation ou une fraction de la coupe naphta comprenant des composés ayant un point d'ébullition inférieur ou égal à 175°C issue de l'étape e) de fractionnement est envoyée vers l'étape a) d'hydrogénation sélective et/ou l'étape b) d'hydrotraitement.

Selon une variante, la quantité du flux de recycle des étapes f) et/ou g) est ajustée de sorte que le rapport pondéral entre le flux de recycle et la charge comprenant une huile de pyrolyse de plastiques est inférieur ou égal à 10.

Selon une variante, un flux contenant une amine est injecté en amont de l'étape a).

Selon une variante, le procédé comprend une étape a0) de prétraitement de la charge comprenant une huile de pyrolyse de plastiques, ladite étape de prétraitement étant mise en oeuvre en amont de l'étape a) d'hydrogénation sélective et comprend une étape de filtration et/ou une étape d'un lavage à l'eau et/ou une étape d'adsorption.

Selon une variante, la section réactionnelle de l'étape a) ou b) met en oeuvre aux moins deux réacteurs fonctionnant en mode permutable.

Selon une variante, ledit catalyseur d'hydrogénation sélective comprend un support choisi parmi l'alumine, la silice, les silices-alumines, la magnésie, les argiles et leurs mélanges et une fonction hydro-déshydrogénante comprenant soit au moins un élément du groupe VIII et au moins un élément du groupe VIB, soit au moins un élément du groupe VIII.

Selon une variante, ledit au moins un catalyseur d'hydrotraitement comprend un support choisi dans le groupe constitué par l'alumine, la silice, les silices-alumines, la magnésie, les argiles et leurs mélanges, et une fonction hydro-déshydrogénante comprenant au moins un élément du groupe VIII et/ou au moins un élément du groupe VIB.

Selon une variante, ledit catalyseur d'hydrocraquage comprend un support choisi parmi les alumines halogénées, les combinaisons d'oxydes de bore et d'aluminium, les silice-alumines amorphes et les zéolithes et une fonction hydro-déshydrogénante comprenant au moins un métal du groupe VIB choisi parmi le chrome, le molybdène et le tungstène, seul ou en mélange, et/ou au moins un métal du groupe VIII choisi parmi le fer, le cobalt, le nickel, le ruthénium, le rhodium, le palladium et le platine.

Selon cette variante, ladite zéolithe est choisie parmi les zéolithes Y, seules ou en combinaison, avec d'autres zéolithes parmi les zéolithes beta, ZSM-12, IZM-2, ZSM-22, ZSM-23, SAPO-11, ZSM-48, ZBM-30, seules ou en mélange.

Selon une variante, l'effluent hydrocarboné issu de l'étape d) de séparation, ou au moins l'un des deux flux hydrocarboné(s) liquides issu(s) de l'étape e) optionnelle, est en tout ou partie envoyé vers une étape h) de vapocraquage réalisée dans au moins un four de pyrolyse à une température comprise entre 700 et 900°C et à une pression comprise entre 0,05 et 0,3 MPa relatif.

Selon une variante, la coupe naphta comprenant des composés ayant un point d'ébullition inférieur ou égal à 175°C C issue de l'étape e) est fractionnée en une coupe naphta lourde comprenant des composés ayant un point d'ébullition entre 80 et 175°C et une coupe naphta légère comprenant des composés ayant un point d'ébullition inférieure à 80°C, au moins une partie de ladite coupe lourde étant envoyée vers un complexe aromatique comportant au moins une étape de reformage du naphta.

Selon cette variante, au moins une partie de_la coupe naphta légère est envoyée dans l'étape h) de vapocraquage.

L'invention concerne également le produit susceptible d'être obtenu par le procédé de traitement selon l'invention.

Selon la présente invention, les pressions sont des pressions absolues, encore notées abs., et sont données en MPa absolus (ou MPa abs.), sauf indication contraire.

Selon la présente invention, les expressions « compris entre ... et ... » et « entre .... et ... » sont équivalentes et signifient que les valeurs limites de l'intervalle sont incluses dans la gamme de valeurs décrite. Si tel n'était pas le cas et que les valeurs limites n'étaient pas incluses dans la gamme décrite, une telle précision sera apportée par la présente invention.

Dans le sens de la présente invention, les différentes plages de paramètre pour une étape donnée tels que les plages de pression et les plages température peuvent être utilisés seul ou en combinaison. Par exemple, dans le sens de la présente invention, une plage de valeurs préférées de pression peut être combinée avec une plage de valeurs de température plus préférées.

Dans la suite, des modes de réalisation particuliers et/ou préférés de l'invention peuvent être décrits. Ils pourront être mis en oeuvre séparément ou combinés entre eux, sans limitation de combinaison lorsque c'est techniquement réalisable.

Dans la suite, les groupes d'éléments chimiques sont donnés selon la classification CAS (CRC Handbook of Chemistry and Physics, éditeur CRC press, rédacteur en chef D.R. Lide, 81ème édition, 2000-2001). Par exemple, le groupe VIII selon la classification CAS correspond aux métaux des colonnes 8, 9 et 10 selon la nouvelle classification IUPAC.

La teneur en métaux est mesurée par fluorescence X.

## DESCRIPTION DETAILLEE

### La charge

Selon l'invention, une « huile de pyrolyse de plastiques » est une huile, avantageusement sous forme liquide à température ambiante, issue de la pyrolyse de plastiques, de préférence de déchets plastiques provenant notamment de filières de collecte et de tri. Elle comprend en particulier un mélange de composés hydrocarbonés, notamment des paraffines, des mono- et/ou di-oléfines, des naphtènes et des aromatiques, ces composés hydrocarbonés ayant de préférence un point d'ébullition inférieur à 700°C et de manière préférée inférieur à 550°C. L'huile de pyrolyse de plastiques peut comprendre, et le plus souvent comprend, en outre des impuretés comme des métaux, notamment du silicium et du fer, des composés halogénés, notamment des composés chlorés. Ces impuretés peuvent être présentes dans l'huiles de pyrolyse de plastiques à des teneurs élevées, par exemple jusqu'à 350 ppm poids ou encore 700 ppm poids voire 1000 ppm poids d'éléments halogène apportés par des composés halogénés, jusqu'à 100 ppm poids, voire 200 ppm poids d'éléments métalliques ou semi-métalliques. Les métaux alcalins, les alcalino terreux, les métaux de transition, les métaux pauvres et les métalloïdes peuvent être assimilés aux contaminants de nature métallique, appelés métaux ou éléments métalliques ou semi métalliques. De manière particulière, les métaux ou éléments métalliques ou semi métalliques, éventuellement contenus dans les huiles issues de la pyrolyse des déchets plastiques, comprennent du silicium, du fer ou ces deux éléments. L'huile de pyrolyse de plastiques peut également comprendre d'autres impuretés comme des hétéroéléments apportés notamment par des composés soufrés, des composés oxygénés et/ou des composés azotés, à des teneurs généralement inférieures à 10000 ppm poids d'hétéroéléments et de préférence inférieures à 4000 ppm poids d'hétéroéléments.

La charge du procédé selon l'invention comprend au moins une huile de pyrolyse de plastiques. Ladite charge peut être constituée uniquement d'huile(s) de pyrolyse de plastiques. De préférence, ladite charge comprend au moins 50% poids, de manière préférée entre 75 et 100% poids, d'huile de pyrolyse de plastiques, c'est-à-dire de préférence entre 50 et 100% poids, de manière préférée entre 70% et 100% poids de d'huile de pyrolyse de plastiques. La charge du procédé selon l'invention peut comprendre, entre autre une ou des huile(s) de pyrolyse de plastiques, une charge pétrolière conventionnelle ou une charge issue de la conversion de la biomasse qui est alors co-traitée avec l'huile de pyrolyse de plastiques de la charge.

L'huile de pyrolyse de plastiques peut être issue d'un traitement de pyrolyse thermique, catalytique ou encore être préparée par hydropyrolyse (pyrolyse en présence d'un catalyseur et d'hydrogène).

**Prétraitement (optionnel)**

Ladite charge comprenant une huile de pyrolyse de plastiques peut avantageusement être prétraitée dans une étape optionnelle de prétraitement a0), préalablement à l'étape a) d'hydrogénation sélective, pour obtenir une charge prétraitée qui alimente l'étape a).

Cette étape optionnelle de prétraitement a0) permet de diminuer la quantité de contaminants, en particulier la quantité de silicium, éventuellement présents dans la charge comprenant une huile de pyrolyse de plastiques. Ainsi, une étape optionnelle a0) de prétraitement de la charge comprenant une huile de pyrolyse de plastiques est avantageusement réalisée en particulier lorsque ladite charge comprend plus de 50 ppm poids, notamment plus de 20 ppm poids, plus particulièrement plus de 10 ppm poids, voire plus de 5 ppm poids d'éléments métalliques, et en particulier lorsque ladite charge comprend plus de 20 ppm poids de silicium, plus particulièrement plus de 10 ppm poids, voire plus de 5 ppm poids et encore plus particulièrement plus de 1,0 ppm poids de silicium.

Ladite étape optionnelle de prétraitement a0) peut être mise en oeuvre par n'importe quelle méthode connue par l'homme du métier permettant de diminuer la quantité de contaminants. Elle peut notamment comprendre une étape de filtration et/ou une étape d'un lavage à l'eau et/ou une étape d'adsorption.

Selon une variante, ladite étape optionnelle de prétraitement a0) est mise en oeuvre dans une section d'adsorption opérée en présence d'au moins un adsorbant. Ladite étape optionnelle de prétraitement a0) est mise en oeuvre à une température entre 0 et 150°C, de préférence entre 5 et 100°C, et à une pression entre 0,15 et 10,0 MPa abs, de préférence entre 0,2 et 1,0 MPa abs. La section d'adsorption est opérée avantageusement en présence d'au moins un adsorbant, de préférence de type alumine, ayant une surface spécifique supérieure ou égale à 100 m$^2$/g, de préférence supérieure ou égale à 200 m$^2$/g. La surface spécifique dudit au moins adsorbant est avantageusement inférieure ou égale à 600 m$^2$/g, en particulier inférieure ou égale à 400 m$^2$/g. La surface spécifique de l'adsorbant est une surface mesurée par la méthode BET, c'est-à-dire la surface spécifique déterminée par adsorption d'azote conformément à la norme ASTM D 3663-78 établie à partir de la méthode BRUNAUER-EMMETT-TELLER décrite dans le périodique 'The Journal of the American Chemical Society", 6Q, 309 (1938).

Avantageusement, ledit adsorbant comprend moins de 1% poids d'éléments métalliques, de préférence est exempt d'éléments métalliques. Par éléments métalliques de l'adsorbant, il faut entendre les éléments des groupes 6 à 10 du tableau périodique des éléments (nouvelle classification IUPAC).

Ladite section d'adsorption de l'étape optionnelle a0) comprend au moins une colonne d'adsorption, de préférence comprend au moins deux colonnes d'adsorption, préférentiellement entre deux et quatre colonnes d'adsorption, contenant ledit adsorbant. Lorsque la section d'adsorption comprend deux colonnes d'adsorption, un mode de fonctionnement peut être un fonctionnement appelé « en swing », selon le terme anglo-saxon consacré, dans lequel l'une des colonnes est en ligne, c'est-à-dire en fonctionnement, tandis que l'autre colonne est en réserve. Lorsque l'absorbant de la colonne en ligne est usé, cette colonne est isolée tandis que la colonne en réserve est mise en ligne, c'est-à-dire en fonctionnement. L'absorbant usé peut être ensuite régénéré *in situ* et/ou remplacé par de l'absorbant frais pour que la colonne le contenant puisse à nouveau être remise en ligne une fois que l'autre colonne aura été isolée.

Un autre mode de fonctionnement est d'avoir au moins deux colonnes fonctionnant en série. Lorsque l'absorbant de la colonne placée en tête est usé, cette première colonne est isolée et l'absorbant usée est soit régénéré *in situ* ou remplacé par de l'absorbant frais. La colonne est ensuite remise en ligne en dernière position et ainsi de suite. Ce fonctionnement est appelé mode permutable, ou selon le terme anglais « PRS » pour Permutable Reactor System ou encore « lead and lag » selon le terme anglo-saxon consacré. L'association d'au moins deux colonnes d'adsorption permet de palier à l'empoisonnement et/ou au colmatage possible et éventuellement rapide de l'adsorbant sous l'action conjointe des contaminants métalliques, des dioléfines, des gommes issues des dioléfines et des insolubles éventuellement présents dans l'huile de pyrolyse de plastiques à traiter. La présence d'au moins deux colonnes d'adsorption facilite en effet le remplacement et/ou la régénération de l'adsorbant, avantageusement sans arrêt de l'unité de prétraitement, voire du procédé, permettant ainsi de diminuer les risques de colmatage et donc d'éviter l'arrêt de l'unité dû au colmatage, de maitriser les coûts et de limiter la consommation d'adsorbant.

Ladite étape optionnelle a0) de prétraitement peut également être éventuellement alimentée par au moins une fraction d'un flux de recycle, avantageusement issu de l'étape g) du procédé, en mélange ou séparément de la charge comprenant une huile de pyrolyse de plastiques.

Ladite étape optionnelle a0) de prétraitement permet ainsi d'obtenir une charge prétraitée qui alimente ensuite l'étape a) d'hydrogénation sélective.

**Etape a) d'hydrogénation sélective**

Selon l'invention, le procédé comprend une étape a) d'hydrogénation sélective de la charge comprenant une huile de pyrolyse de plastiques réalisée en présence d'hydrogène, dans des conditions de pression en hydrogène et de température permettant de maintenir ladite charge en phase liquide et avec une quantité d'hydrogène soluble juste nécessaire à une hydrogénation sélective des dioléfines présentes dans l'huile de pyrolyse de plastiques. L'hydrogénation sélective des dioléfines en phase liquide permet ainsi d'éviter ou au moins de limiter la formation de « gommes », c'est-à-dire la polymérisation des dioléfines et donc la formation d'oligomères et polymères, pouvant boucher la section réactionnelle de l'étape b) d'hydrotraitement. Ladite étape a) d'hydrogénation sélective permet d'obtenir un effluent hydrogéné, c'est-à-dire un effluent à teneur réduite en oléfines, en particulier en dioléfines, de préférence exempt de dioléfines.

Selon l'invention, ladite étape a) d'hydrogénation sélective est mise en oeuvre dans une section réactionnelle alimentée au moins par ladite charge comprenant une huile de pyrolyse de plastiques, ou par la charge prétraitée issue de l'éventuelle étape a0) de prétraitement, et un flux gazeux comprenant de l'hydrogène ($H_2$). Eventuellement, la section réactionnelle de ladite étape a) peut également être alimentée en outre par au moins une fraction d'un flux de recycle, avantageusement issu de l'étape d) ou de l'étape g) optionnelle, soit en mélange avec ladite charge, éventuellement prétraitée, soit séparément de la charge, éventuellement prétraitée, avantageusement directement en entrée d'au moins un des réacteurs de la section réactionnelle de l'étape a). L'introduction d'au moins une fraction dudit flux de recycle dans la section réactionnelle de l'étape a) d'hydrogénation sélective permet avantageusement de diluer les impuretés de la charge, éventuellement prétraitée, et de contrôler la température notamment dans ladite section réactionnelle.

Ladite section réactionnelle met en oeuvre une hydrogénation sélective, de préférence en lit fixe, en présence d'au moins un catalyseur d'hydrogénation sélective, avantageusement à une température entre 100 et 280°C, de préférence entre 120 et 260°C, de manière préférée entre 130 et 250°C, une pression partielle d'hydrogène entre 1,0 et 10,0 MPa abs, de manière préférée entre 1,5 et 8,0 MPa abs et à une vitesse volumique horaire (VVH) entre 0,3 et 10,0 $h^{-1}$, de manière préférée entre 0,5 et 5,0 $h^{-1}$. La vitesse volumique horaire (VVH) est définie ici comme le ratio entre le débit volumique horaire de la charge comprenant l'huile de pyrolyse de plastiques, éventuellement prétraitée, par le volume de catalyseur(s). La quantité du flux gazeux comprenant de l'hydrogène ($H_2$), alimentant ladite section réactionnelle de l'étape a), est avantageusement telle que la couverture en hydrogène est comprise entre 1 et 200 $Nm^3$ d'hydrogène par $m^3$ de charge ($Nm^3/m^3$), de préférence entre 1 et 50 $Nm^3$ d'hydrogène par $m^3$ de charge ($Nm^3/m^3$), de manière préférée entre 5 et 20 $Nm^3$ d'hydrogène par $m^3$ de charge ($Nm^3/m^3$). La couverture en hydrogène est définie comme le rapport du débit volumique d'hydrogène pris dans les conditions normales de température et pression par rapport au débit volumique de charge « fraîche », c'est-à-dire de la charge à traiter, éventuellement prétraitée, sans tenir compte de l'éventuelle fraction recyclée, à 15°C (en normaux $m^3$, noté $Nm^3$, de $H_2$ par $m^3$ de charge). Le flux gazeux comprenant de l'hydrogène, qui alimente la section réactionnelle de l'étape a), peut être constitué d'un appoint en hydrogène et/ou d'hydrogène recyclé issu en particulier de l'étape d) de séparation.

Avantageusement, la section réactionnelle de ladite étape a) comprend entre 1 et 5 réacteurs. Selon un mode de réalisation particulier de l'invention, la section réactionnelle comprend entre 2 et 5 réacteurs, qui fonctionnent en mode permutable, appelé selon le terme anglais « PRS » pour Permutable Reactor System ou encore « lead and lag ». L'association d'au moins deux réacteurs en mode PRS permet d'isoler un réacteur, de décharger le catalyseur usé, de recharger le réacteur en catalyseur frais et remettre en service ledit réacteur sans arrêt du procédé. La technologie PRS est décrite, en particulier, dans le brevet FR2681871.

Avantageusement, des internes de réacteurs, par exemple de type plateaux filtrants, peuvent être utilisés pour prévenir le bouchage du(des) réacteur(s). Un exemple de plateau filtrant est décrit dans le brevet FR3051375.

Avantageusement, ledit au moins catalyseur d'hydrogénation sélective comprend un support, de préférence minéral, et une fonction hydro-déshydrogénante.

Selon une variante, la fonction hydro-déshydrogénante comprend en particulier au moins un élément du groupe VIII, de préférence choisi parmi le nickel et le cobalt, et au moins un élément du groupe VIB, de préférence choisi parmi le molybdène et le tungstène. Selon cette variante, la teneur totale en oxydes des éléments métalliques des groupes VIB et VIII est de préférence comprise entre 1% et 40% en poids, préférentiellement de 5% à 30% en poids par rapport au poids total du catalyseur. Le rapport pondéral exprimé en oxyde métallique entre le métal (ou les métaux) du groupe VIB par rapport au métal (ou aux métaux) du groupe VIII est de préférence compris entre 1 et 20, et de manière préférée entre 2 et 10.

Selon cette variante, la section réactionnelle de ladite étape a) comprend par exemple un catalyseur d'hydrogénation sélective comprenant entre 0,5% et 12% en poids de nickel, de préférence entre 1% et 10% en poids de nickel (exprimé en oxyde de nickel NiO par rapport au poids dudit catalyseur), et entre 1% et 30% en poids de molybdène, de préférence entre 3% et 20% en poids de molybdène (exprimé en oxyde de molybdène $MoO_3$ par rapport au poids dudit catalyseur) sur un support de préférence minéral, de préférence sur un support d'alumine.

Selon une autre variante, la fonction hydro-déshydrogénante comprend, et est de préférence constituée d'au moins un élément du groupe VIII, de préférence du nickel. Selon cette variante, la teneur en oxydes de nickel est de préférence comprise entre 1 et 50 % en poids, de préférence entre 10% et 30% en poids par rapport au poids dudit catalyseur. Ce type de catalyseur est de préférence utilisé sous sa forme réduite, sur un support de préférence minéral, de préférence sur un support d'alumine.

Le support dudit au moins catalyseur d'hydrogénation sélective est de préférence choisi parmi l'alumine, la silice, les silices-alumines, la magnésie, les argiles et leurs mélanges. Ledit support peut renfermer des composés dopants, notamment des oxydes choisis parmi l'oxyde de bore, en particulier le trioxyde de bore, la zircone, la cérine, l'oxyde de titane, l'anhydride phosphorique et un mélange de ces oxydes. De préférence, ledit au moins catalyseur d'hydrogénation sélective comprend un support d'alumine, éventuellement dopé avec du phosphore et éventuellement du bore. Lorsque l'anhydride phosphorique $P_2O_5$ est présent, sa concentration est inférieure à 10% en poids par rapport au poids de l'alumine et avantageusement d'au moins 0,001 % poids par rapport au poids total de l'alumine. Lorsque le trioxyde de bore $B_2O_5$ est présent, sa concentration est inférieure à 10% en poids par rapport au poids de l'alumine et avantageusement d'au moins 0,001 % par rapport au poids total de l'alumine. L'alumine utilisée peut être par exemple une alumine γ (gamma) ou η (éta).

Ledit catalyseur d'hydrogénation sélective est par exemple sous forme d'extrudés.

De manière très préférée, afin d'hydrogéner les dioléfines le plus sélectivement possible, l'étape a) peut mettre en oeuvre en plus des catalyseurs d'hydrogénation sélective décrits ci-dessus en outre au moins un catalyseur d'hydrogénation sélective utilisé dans l'étape a) comprenant moins de 1% en poids de nickel et au moins 0,1 % poids de nickel, de préférence 0,5% poids de nickel, exprimé en oxyde de nickel NiO par rapport au poids dudit catalyseur, et moins de 5% en poids de molybdène et au moins 0,1 % poids de molybdène, de préférence 0,5% poids de molybdène, exprimé en oxyde de molybdène $MoO_3$ par rapport au poids dudit catalyseur, sur un support d'alumine. Ce catalyseur peu chargé en métaux est de préférence mis en amont des catalyseurs d'hydrogénation sélective décrits ci-dessus.

Eventuellement, la charge qui comprend une huile de pyrolyse de plastiques, éventuellement prétraitée, et/ou éventuellement mélangée préalablement avec au moins une fraction d'un flux de recycle, avantageusement issu de l'étape d) ou de l'étape g) optionnelle, peut être mélangée avec le flux gazeux comprenant de l'hydrogène préalablement à son introduction dans la section réactionnelle.

Ladite charge, éventuellement prétraitée, et/ou éventuellement mélangée avec au moins une fraction du flux de recycle, avantageusement issu de l'étape d) ou de l'étape g) optionnelle, et/ou éventuellement en mélange avec le flux gazeux, peut également être chauffée avant son introduction dans la section réactionnelle de l'étape a), par exemple par échange de chaleur notamment avec l'effluent d'hydrotraitement de l'étape b), pour atteindre une température proche de la température mise en oeuvre dans la section réactionnelle qu'elle alimente.

La teneur en impuretés, en particulier en dioléfines, de l'effluent hydrogéné obtenu à l'issue de l'étape a) est réduite par rapport à celle des mêmes impuretés, en particulier des dioléfines, comprises dans la charge du procédé. L'étape a) d'hydrogénation sélective permet généralement de convertir au moins 90% et de préférence au moins 99% des dioléfines contenues dans la charge initiale. L'étape a) permet également l'élimination, au moins en partie, d'autres contaminants, comme par exemple le silicium. L'effluent hydrogéné, obtenu à l'issue de l'étape a) d'hydrogénation sélective, est envoyé, de préférence directement, vers l'étape b) d'hydrotraitement. Lorsqu'au moins une fraction du flux de recycle issu de l'étape g) éventuelle est introduite, l'effluent hydrogéné obtenu à l'issue de l'étape a) d'hydrogénation sélective comprend donc, en plus de la charge convertie, ladite ou lesdites fraction(s) du flux de recycle.

**Etape b) d'hydrotraitement**

Selon l'invention, le procédé de traitement comprend une étape b) d'hydrotraitement, avantageusement en lit fixe, dudit effluent hydrogéné issu de l'étape a), éventuellement en mélange avec au moins une fraction d'un flux de recycle, avantageusement issu de l'étape d) ou des étapes f) et/ou g) optionnelles, en présence d'hydrogène et d'au moins un catalyseur d'hydrotraitement, pour obtenir un effluent d'hydrotraitement.

Avantageusement, l'étape b) met en oeuvre les réactions d'hydrotraitement bien connues de l'homme du métier, et plus particulièrement des réactions d'hydrogénation des oléfines, des aromatiques, d'hydrodémétallation, d'hydrodésulfuration, d'hydrodéazotation, etc.

Avantageusement, ladite étape b) est mise en oeuvre dans une section réactionnelle d'hydrotraitement comprenant au moins un, de préférence entre un et cinq, réacteur(s) à lit fixe ayant n lits catalytiques, n étant un nombre entier supérieur ou égal à un, de préférence compris entre un et dix, de manière préférée compris entre deux et cinq, le(s)dit(s) lit(s) comprenant chacun au moins un, et de préférence pas plus de dix, catalyseur(s) d'hydrotraitement. Lorsqu'un réacteur comprend plusieurs lits catalytiques, c'est-à-dire au moins deux, de préférence entre deux et dix, de manière préférée entre deux et cinq lits catalytiques, lesdits lits catalytiques sont disposés en série dans ledit réacteur.

Ladite section réactionnelle d'hydrotraitement est alimentée au moins par ledit effluent hydrogéné issu de l'étape a) et un flux gazeux comprenant de l'hydrogène, avantageusement au niveau du premier lit catalytique du premier réacteur en fonctionnement.

Ladite section réactionnelle d'hydrotraitement de l'étape b) peut également être alimentée par au moins une fraction du flux de recycle, avantageusement issu de l'étape d) ou des étapes f) et/ou g) optionnelles. La(les)dite(s) fraction(s) dudit flux de recycle ou la totalité du flux de recycle peu(ven)t être introduite(s) dans ladite section réactionnelle d'hydrotraitement en mélange avec l'effluent hydrogéné issu de l'étape a) ou séparément. La(les)dite(s) fraction(s) dudit flux de recycle ou la totalité du flux de recycle peu(ven)t être introduite(s) dans ladite section réactionnelle d'hydrotraitement au niveau d'un ou de plusieurs lits catalytiques de ladite section réactionnelle d'hydrotraitement de l'étape b). L'introduction d'au moins une fraction dudit flux de recycle permet avantageusement de diluer les impuretés encore présentes dans l'effluent hydrogéné et de contrôler la température, en particulier de limiter l'augmentation de température, dans le ou les lit(s) catalytique(s) de la section réactionnelle d'hydrotraitement qui met en oeuvre des réactions fortement exothermiques.

Avantageusement, ladite section réactionnelle d'hydrotraitement est mise en oeuvre à une pression équivalente à celle utilisée dans la section réactionnelle de l'étape a) d'hydrogénation sélective, mais à une plus haute température que celle de la section réactionnelle de l'étape a) d'hydrogénation sélective. Ainsi, ladite section réactionnelle d'hydrotraitement est avantageusement mise en oeuvre à une température d'hydrotraitement entre 250 et 430°C, de préférence entre 280 et 380°C, à une pression partielle d'hydrogène entre 1,0 et 10,0 MPa abs. et à une vitesse volumique horaire (VVH) entre 0,1 et 10,0 h$^{-1}$, de préférence entre 0,1 et 5,0 h$^{-1}$, préférentiellement entre 0,2 et 2,0 h$^{-1}$, de manière préférée entre 0,2 et 0,8 h$^{-1}$. Selon l'invention, la « température d'hydrotraitement » correspond à une température moyenne dans la section réactionnelle d'hydrotraitement de l'étape b). En particulier, elle correspond à la Weight Average Bed Température (WABT) selon le terme anglo-saxon consacré, bien connue de l'Homme du métier. La température d'hydrotraitement est avantageusement déterminée en fonction des systèmes catalytiques, des équipements, de la configuration de ceux-ci, utilisés. Par exemple, la température d'hydrotraitement (ou WABT) est calculée de la manière suivante :

$$WABT = (T_{entrée} + 2x\ T_{sortie})/3$$

avec $T_{entrée}$ : la température de l'effluent hydrogéné en entrée de la section réactionnelle d'hydrotraitement, $T_{sortie}$ : la température de l'effluent en sortie de section réactionnelle d'hydrotraitement.

La vitesse volumique horaire (VVH) est définie ici comme le ratio entre le débit volumique horaire de l'effluent hydrogéné issu de l'étape a) par volume de catalyseur(s). La couverture en hydrogène dans l'étape b) est avantageusement comprise entre 50 et 1000 Nm$^3$ d'hydrogène par m$^3$ de charge fraîche qui alimente l'étape a), et de préférence entre 50 et 500 Nm$^3$ d'hydrogène par m$^3$ de charge fraîche qui alimente l'étape a), de manière préférée entre 100 et 300 Nm$^3$ d'hydrogène par m$^3$ de charge fraîche qui alimente l'étape a). La couverture en hydrogène est définie ici comme le rapport du débit volumique d'hydrogène pris dans les conditions normales de température et pression par rapport au débit volumique de charge fraîche qui alimente l'étape a), c'est-à-dire de charge comprenant une huile de pyrolyse de plastiques, ou par la charge éventuellement prétraitée, qui alimente l'étape a) (en normaux m$^3$ , noté Nm$^3$, de H$_2$ par m$^3$ de charge fraîche). L'hydrogène peut être constitué d'un appoint et/ou d'hydrogène recyclé issu en particulier de l'étape d) de séparation.

De préférence, un flux gazeux supplémentaire comprenant de l'hydrogène est avantageusement introduit en entrée de chaque réacteur, en particulier fonctionnant en série, et/ou en entrée de chaque lit catalytique à partir du second lit catalytique de la section réactionnelle d'hydrotraitement. Ces flux gazeux supplémentaires sont appelés encore flux de refroidissement. Ils permettent de contrôler la température dans le réacteur d'hydrotraitement dans lequel les réactions mises en oeuvre sont généralement très exothermiques.

Avantageusement, ledit catalyseur d'hydrotraitement utilisé dans ladite étape b) peut être choisi parmi des catalyseurs connus d'hydrodémétallation, d'hydrotraitement, de captation du silicium, utilisés notamment pour le traitement des coupes pétrolières, et leurs combinaisons. Des catalyseurs d'hydrodémétallation connus sont par exemple ceux décrits dans les brevets EP 0113297, EP 0113284, US 5221656, US 5827421, US 7119045, US 5622616 et US 5089463. Des catalyseurs d'hydrotraitement connus sont par exemple ceux décrits dans les brevets EP 0113297, EP 0113284, US 6589908, US 4818743 ou US 6332976. Des catalyseurs de captation du silicium connus sont par exemple ceux décrits dans les demandes de brevets CN 102051202 et US 2007/080099.

En particulier, ledit catalyseur d'hydrotraitement comprend un support, de préférence minéral, et au moins un élément métallique ayant une fonction hydro-déshydrogénante. Ledit élément métallique ayant une fonction hydro-déshydrogénante comprend avantageusement au moins un élément du groupe VIII, de préférence choisi dans le groupe constitué par le nickel et le cobalt, et/ou au moins un élément du groupe VIB, de préférence choisi dans le groupe constitué par le molybdène et le tungstène. La teneur totale en oxydes des éléments métalliques des groupes VIB et VIII est de préférence entre 0,1% et 40% en poids, préférentiellement de 5% à 35% en poids, par rapport au poids total du catalyseur. Le rapport pondéral exprimé en oxyde métallique entre le métal (ou les métaux) du groupe VIB par rapport au métal (ou aux métaux) du groupe VIII est de préférence compris entre 1,0 et 20, de manière préférée entre 2,0 et 10. Par exemple, la section réactionnelle d'hydrotraitement de l'étape b) du procédé comprend un catalyseur d'hydrotraitement comprenant entre 0,5% et 10% en poids de nickel, de préférence entre 1% et 8% en poids de nickel, exprimé en oxyde de nickel NiO par rapport au poids total du catalyseur d'hydrotraitement, et entre 1,0% et 30% en poids de molybdène, de préférence entre 3,0% et 29% en poids de molybdène, exprimé en oxyde de molybdène $MoO_3$ par rapport au poids total du catalyseur d'hydrotraitement, sur un support minéral.

Le support dudit catalyseur d'hydrotraitement est avantageusement choisi parmi l'alumine, la silice, les silices-alumines, la magnésie, les argiles et leurs mélanges. Ledit support peut en outre renfermer des composés dopants, notamment des oxydes choisis parmi l'oxyde de bore, en particulier le trioxyde de bore, la zircone, la cérine, l'oxyde de titane, l'anhydride phosphorique et un mélange de ces oxydes. De préférence, ledit catalyseur d'hydrotraitement comprend un support d'alumine, de manière préférée un support d'alumine dopé avec du phosphore et éventuellement du bore. Lorsque l'anhydride phosphorique $P_2O_5$ est présent, sa concentration est inférieure à 10% en poids par rapport au poids de l'alumine et avantageusement d'au moins 0,001 % poids par rapport au poids total de l'alumine. Lorsque le trioxyde de bore $B_2O_5$ est présent, sa concentration est inférieure à 10% en poids par rapport au poids de l'alumine et avantageusement d'au moins 0,001 % par rapport au poids total de l'alumine. L'alumine utilisée peut être par exemple une alumine $\gamma$ (gamma) ou $\eta$ (éta).

Ledit catalyseur d'hydrotraitement est par exemple sous forme d'extrudés.

Avantageusement, ledit catalyseur d'hydrotraitement utilisé dans l'étape b) du procédé présente une surface spécifique supérieure ou égale à 250 m$^2$/g, de préférence supérieure ou égale à 300 m$^2$/g. La surface spécifique dudit catalyseur d'hydrotraitement est avantageusement inférieure ou égale à 800 m$^2$/g, de préférence inférieure ou égale à 600 m$^2$/g, en particulier inférieure ou égale à 400 m$^2$/g. La surface spécifique du catalyseur d'hydrotraitement est mesurée par la méthode BET, c'est-à-dire la surface spécifique déterminée par adsorption d'azote conformément à la norme ASTM D 3663-78 établie à partir de la méthode BRUNAUER-EMMETT-TELLER décrite dans le périodique 'The Journal of the American Chemical Society", 6Q, 309 (1938). Une telle surface spécifique permet d'améliorer encore l'élimination des contaminants, en particulier des métaux comme le silicium.

Selon un autre aspect de l'invention, le catalyseur d'hydrotraitement tel que décrit plus haut comprend en outre un ou plusieurs composés organiques contenant de l'oxygène et/ou de l'azote et/ou du soufre. Un tel catalyseur est souvent désigné par le terme "catalyseur additivé". Généralement, le composé organique est choisi parmi un composé comportant une ou plusieurs fonctions chimiques choisies parmi une fonction carboxylique, alcool, thiol, thioéther, sulfone, sulfoxyde, éther, aldéhyde, cétone, ester, carbonate, amine, nitrile, imide, oxime, urée et amide ou encore les composés incluant un cycle furanique ou encore les sucres.

L'étape b) d'hydrotraitement permet avantageusement un traitement optimisé de l'effluent hydrogéné issu de l'étape a). Elle permet, en particulier, de maximiser l'hydrogénation des liaisons insaturées des composés oléfiniques présents dans l'effluent hydrogéné issu de l'étape a), l'hydrodémétallation dudit effluent hydrogéné et la captation des métaux, notamment le silicium, encore présents dans l'effluent hydrogéné. L'étape b) d'hydrotraitement permet également l'hydrodéazotation (HDN) de l'effluent hydrogéné, c'est-à-dire la conversion des espèces azotés encore présentes dans l'effluent hydrogéné. De préférence, la teneur en azote de l'effluent d'hydrotraitement à l'issue de l'étape b) est inférieure ou égale à 10 ppm en poids.

Dans un mode de réalisation préféré de l'invention, ladite section réactionnelle d'hydrotraitement comprend plusieurs réacteurs à lit fixe, préférentiellement entre deux et cinq, très préférentiellement entre deux et quatre, réacteurs à lit fixe, ayant chacun n lits catalytiques, n étant un nombre entier supérieur ou égal à un, de préférence compris entre un et dix, de manière préférée compris entre deux et cinq, et fonctionnant avantageusement en série et/ou en parallèle et/ou en mode permutable (ou PRS) et/ou en mode « swing ». Les différents modes de fonctionnement éventuels, mode PRS (ou lead and lag) et mode swing, et sont bien connus de l'Homme du métier et sont avantageusement définis plus haut. L'avantage d'une section réactionnelle d'hydrotraitement comprenant plusieurs réacteurs réside dans un traitement optimisé de l'effluent hydrogéné, tout en permettant de diminuer les risques de colmatage du ou des lits catalytiques et donc d'éviter l'arrêt de l'unité dû au colmatage.

Selon un mode de réalisation très préféré de l'invention, ladite section réactionnelle d'hydrotraitement comprend, de préférence consiste en :

- (b1) deux réacteurs à lit fixe fonctionnant en mode swing ou permutable, de préférence en mode PRS, chacun des deux réacteurs ayant de préférence un lit catalytique comprenant avantageusement un catalyseur d'hydrotraitement de préférence choisi parmi des catalyseurs connus d'hydrodémétallation, de captation du silicium et leurs combinaisons, et

- (b2) au moins un réacteur à lit fixe, de préférence un réacteur, situé en aval des deux réacteurs (b1), et avantageusement fonctionnant en série des deux réacteurs (b1), ledit réacteur (b2) à lit fixe ayant entre 1 et 5 lits catalytiques disposés en série et comprenant chacun entre un et dix catalyseur(s) d'hydrotraitement dont au moins un desdits catalyseurs d'hydrotraitement comprend avantageusement un support et au moins un élément métallique comprenant de préférence au moins un élément du groupe VIII, de préférence choisi parmi le nickel et le cobalt, et/ou au moins un élément du groupe VIB, de préférence choisi parmi le molybdène et le tungstène.

Eventuellement, l'étape b) peut mettre en oeuvre une section de chauffe située en amont de la section réactionnelle d'hydrotraitement et dans laquelle l'effluent hydrogéné issu de l'étape a) est chauffé pour atteindre une température adaptée pour l'hydrotraitement, c'est-à-dire une température comprise entre 250 et 430°C. Ladite éventuelle section de chauffe peut ainsi comprendre un ou plusieurs échangeurs, permettant de préférence un échange de chaleur entre l'effluent hydrogéné et l'effluent d'hydrotraitement, et/ou un four de préchauffe.

Avantageusement, l'étape b) d'hydrotraitement permet l'hydrogénation totale des oléfines présentes dans la charge initiale et celles éventuellement obtenues après l'étape a) d'hydrogénation sélective, mais aussi la conversion au moins en partie d'autres impuretés présentes dans la charge, comme les composés aromatiques, les composés métalliques, les composés soufrés, les composés azotés, les composés halogénés (notamment les composés chlorés), les composés oxygénés. L'étape b) peut également permettre de réduire encore la teneur en contaminants, comme celle des métaux, en particulier la teneur en silicium.

## Etape c) d'hydrocraquage

Selon l'invention, le procédé de traitement comprend une étape c) d'hydrocraquage, avantageusement en lit fixe, dudit effluent hydrotraité issu de l'étape b), éventuellement en mélange avec au moins une fraction d'un flux de recycle avantageusement issu de l'étape d) ou des étapes f) et/ou g) optionnelles, en présence d'hydrogène et d'au moins un catalyseur d'hydrocraquage, pour obtenir un effluent hydrocraqué.

Avantageusement, l'étape c) met en oeuvre les réactions d'hydrocraquage bien connues de l'homme du métier, et permet plus particulièrement de convertir les composés lourds, par exemple des composés ayant un point d'ébullition supérieur à 175°C en composés ayant un point d'ébullition inférieur ou égal à 175°C contenus dans l'effluent hydrotraité issu de l'étape b). D'autres réactions, comme l'hydrogénation des oléfines, des aromatiques, l'hydrodémétallation, l'hydrodésulfuration, l'hydrodéazotation, etc. peuvent se poursuivent.

Avantageusement, ladite étape c) est mise en oeuvre dans une section réactionnelle d'hydrocraquage comprenant au moins un, de préférence entre un et cinq, réacteur(s) à lit fixe ayant n lits catalytiques, n étant un nombre entier supérieur ou égal à un, de préférence compris entre un et dix, de manière préférée compris entre deux et cinq, le(s)dit(s) lit(s) comprenant chacun au moins un, et de préférence pas plus de dix, catalyseur(s) d'hydrocraquage. Lorsqu'un réacteur comprend plusieurs lits catalytiques, c'est-à-dire au moins deux, de préférence entre deux et dix, de manière préférée entre deux et cinq lits catalytiques, lesdits lits catalytiques sont disposés en série dans ledit réacteur.

L'étape b) d'hydrotraitement et l'étape c) d'hydrocraquage peuvent avantageusement être réalisées dans un même réacteur ou dans des réacteurs différents. Dans le cas où elles sont réalisées dans un même réacteur, le réacteur comprend plusieurs lits catalytiques, les premiers lits catalytiques comprenant le ou les catalyseurs d'hydrotraitement et les lits catalytiques suivants comprenant le ou les catalyseurs d'hydrocraquage.

Ladite section réactionnelle d'hydrocraquage est alimentée au moins par ledit effluent hydrotraité issu de l'étape b) et un flux gazeux comprenant de l'hydrogène, avantageusement au niveau du premier lit catalytique du premier réacteur en fonctionnement.

Ladite section réactionnelle d'hydrocraquage de l'étape c) peut également être alimentée par au moins une fraction du flux de recycle avantageusement issu de l'étape d) ou des étapes f) et/ou g) optionnelles. La(les)dite(s) fraction(s) dudit flux de recycle ou la totalité du flux de recycle peu(ven)t être introduite(s) dans ladite section réactionnelle d'hydrocraquage en mélange avec l'effluent hydrotraité issu de l'étape b), ou séparément. La(les)dite(s) fraction(s) dudit flux de recycle ou la totalité du flux de recycle peu(ven)t être introduite(s) dans ladite section réactionnelle d'hydrocraquage au niveau d'un ou de plusieurs lits catalytiques de ladite section réactionnelle d'hydrocraquage de l'étape c).

L'introduction d'au moins une fraction dudit flux de recycle permet avantageusement de diluer les impuretés encore présentes dans l'effluent hydrogéné et de contrôler la température, en particulier de limiter l'augmentation de température, dans le ou les lit(s) catalytique(s) de la section réactionnelle d'hydrocraquage qui met en oeuvre des réactions fortement exothermiques.

Avantageusement, ladite section réactionnelle d'hydrocraquage est mise en oeuvre à une pression équivalente à celle utilisée dans la section réactionnelle de l'étape a) d'hydrogénation sélective ou l'étape b) d'hydrotraitement.

Ainsi, ladite section réactionnelle d'hydrocraquage est avantageusement mise en oeuvre à une température d'hydrotraitement entre 250 et 480°C, de préférence entre 320 et 450°C, à une pression partielle d'hydrogène entre 1,5 et 25,0 MPa abs., de préférence entre 2 et 20 MPa abs, et à une vitesse volumique horaire (VVH) entre 0,1 et 10,0 $h^{-1}$, de préférence entre 0,1 et 5,0 $h^{-1}$, préférentiellement entre 0,2 et 4 $h^{-1}$. Selon l'invention, la « température d'hydrocraquage » correspond à une température moyenne dans la section réactionnelle d'hydrocraquage de l'étape c). En particulier, elle correspond à la Weight Average Bed Température (WABT) selon le terme anglo-saxon consacré, bien connue de l'Homme du métier. La température d'hydrocraquage est avantageusement déterminée en fonction des systèmes catalytiques, des équipements, de la configuration de ceux-ci, utilisés. Par exemple, la température d'hydrocraquage (ou WABT) est calculée de la manière suivante :

$$WABT = (T_{entrée} + 2x\ T_{sortie})/3$$

avec $T_{entrée}$ : la température de l'effluent hydrogéné en entrée de la section réactionnelle d'hydrocraquage, $T_{sortie}$ : la température de l'effluent en sortie de section réactionnelle d'hydrocraquage.

La vitesse volumique horaire (VVH) est définie ici comme le ratio entre le débit volumique horaire de l'effluent hydrogéné issu de l'étape a) par volume de catalyseur(s). La couverture en hydrogène dans l'étape c) est avantageusement comprise entre 80 et 2000 $Nm^3$ d'hydrogène par $m^3$ de charge fraîche qui alimente l'étape a), et de préférence entre 200 et 1800 $Nm^3$ d'hydrogène par $m^3$ de charge fraîche qui alimente l'étape a). La couverture en hydrogène est définie ici comme le rapport du débit volumique d'hydrogène pris dans les conditions normales de température et pression par rapport au débit volumique de charge fraîche qui alimente l'étape a), c'est-à-dire de charge comprenant une huile de pyrolyse de plastiques, ou par la charge éventuellement prétraitée, qui alimente l'étape a) (en normaux $m^3$ , noté $Nm^3$, de $H_2$ par $m^3$ de charge fraîche). L'hydrogène peut être constitué d'un appoint et/ou d'hydrogène recyclé issu en particulier de l'étape d) de séparation.

De préférence, un flux gazeux supplémentaire comprenant de l'hydrogène est avantageusement introduit en entrée de chaque réacteur, en particulier fonctionnant en série, et/ou en entrée de chaque lit catalytique à partir du second lit catalytique de la section réactionnelle d'hydrocraquage. Ces flux gazeux supplémentaires sont appelés encore flux de refroidissement. Ils permettent de contrôler la température dans le réacteur d'hydrocraquage dans lequel les réactions mises en oeuvre sont généralement très exothermiques.

Dans un mode de réalisation permettant de maximiser la production d'une coupe naphta comprenant des composés ayant un point d'ébullition inférieur ou égal à 175°C, les conditions opératoires utilisées dans l'étape c) d'hydrocraquage permettent généralement d'atteindre des conversions par passe de la charge en entrée de l'étape c) d'hydrocraquage, en produits ayant au moins 80% en volume de produits ayant des points d'ébullition inférieurs ou égal à 175°C, de préférence inférieurs à 160°C et de manière préférée inférieurs à 150°C, supérieures à 15% poids et de manière encore plus préférée comprises entre 20 et 95% poids.

L'étape c) d'hydrocraquage_ne permet ainsi forcément de transformer tous les composés ayant un point d'ébullition supérieur à 175°C en composés ayant un point d'ébullition inférieur ou égal à 175°C. Après l'étape de fractionnement e) optionnelle, il peut rester donc une proportion plus ou moins importante de composés ayant un point d'ébullition supérieur à 175°C. Pour augmenter la conversion, au moins une partie de cette coupe non convertie peut être recyclée comme décrit ci-dessous à l'étape f). Une autre partie peut être purgée, comme décrit ci-dessous.

Conformément à l'invention, l'étape c) d'hydrocraquage opère en présence d'au moins un catalyseur d'hydrocraquage.

Le ou les catalyseur(s) d'hydrocraquage utilisé(s) dans l'étape c) d'hydrocraquage sont des catalyseurs classiques d'hydrocraquage connus de l'Homme du métier, de type bifonctionnel associant une fonction acide à une fonction hydro-déshydrogénante et éventuellement au moins une matrice liante. La fonction acide est apportée par des supports de grande surface (150 à 800 $m^2$/g généralement) présentant une acidité superficielle, telles que les alumines halogénées (chlorées ou fluorées notamment), les combinaisons d'oxydes de bore et d'aluminium, les silice-alumines amorphes et les zéolithes. La fonction hydro-déshydrogénante est apportée par au moins un métal du groupe VIB de la classification périodique et/ou au moins un métal du groupe VIII.

De préférence, le ou les catalyseurs d'hydrocraquage utilisés dans l'étape c) comprennent une fonction hydro-déshydrogénante comprenant au moins un métal du groupe VIII choisi parmi le fer, le cobalt, le nickel, le ruthénium, le rhodium, le palladium et le platine, et de préférence parmi le cobalt et le nickel. De préférence, le(s)dit(s) catalyseurs comprennent également au moins un métal du groupe VIB choisi parmi le chrome, le molybdène et le tungstène, seul ou en mélange, et de préférence parmi le molybdène et le tungstène. Des fonctions hydro-déshydrogénantes de type NiMo, NiMoW, NiW sont préférées.

De préférence, la teneur en métal du groupe VIII dans le ou les catalyseur(s) d'hydrocraquage est avantageusement comprise entre 0,5 et 15% poids et de préférence entre 1 et 10% poids, les pourcentages étant exprimés en pourcentage poids d'oxydes par rapport au poids total du catalyseur.

De préférence, la teneur en métal du groupe VIB dans le ou les catalyseur(s) d'hydrocraquage est avantageusement comprise entre 5 et 35% poids, et de préférence entre 10 et 30% poids, les pourcentages étant exprimés en pourcentage poids d'oxydes par rapport au poids total du catalyseur.

Le ou les catalyseur(s) d'hydrocraquage utilisés dans l'étape c) peuvent également comprendre éventuellement au moins un élément promoteur déposé sur le catalyseur et choisi dans le groupe formé par le phosphore, le bore et le silicium, éventuellement au moins un élément du groupe VIIA (chlore, fluor préférés), éventuellement au moins un élément du groupe VIIB (manganèse préféré), et éventuellement au moins un élément du groupe VB (niobium préféré).

De préférence, le ou les catalyseur(s) d'hydrocraquage utilisés dans l'étape c) comprennent au moins une matrice minérale poreuse amorphe ou mal cristallisée de type oxyde choisie parmi les alumines, les silices, les silice-alumines, les aluminates, l'alumine-oxyde de bore, la magnésie, la silice-magnésie, le zircone, l'oxyde de titane, l'argile, seuls ou en mélange, et de préférence les alumines ou les silice-alumines, seules ou en mélange.

De préférence, la silice-alumine contient plus de 50% poids d'alumine, de préférence plus de 60% poids d'alumine.

De préférence, le ou les catalyseur(s) d'hydrocraquage utilisés dans l'étape c) comprennent également éventuellement une zéolithe choisie parmi les zéolithes Y, de préférence parmi les zéolithes USY, seules ou en combinaison, avec d'autres zéolithes parmi les zéolithes beta, ZSM-12, IZM-2, ZSM-22, ZSM-23, SAPO-11, ZSM-48, ZBM-30, seules ou en mélange. De manière préférée la zéolithe est la zéolithe USY seule.

Dans le cas où ledit catalyseur comprend une zéolithe, la teneur en zéolithe dans le ou les catalyseur(s) d'hydrocraquage est avantageusement comprise entre 0,1 et 80% poids, de préférence comprise entre 3 et 70% poids, les pourcentages étant exprimés en pourcentage de zéolithe par rapport au poids total du catalyseur.

Un catalyseur préféré comprend, et est de préférence constitué, d'au moins un métal du groupe VIB et éventuellement d'au moins un métal du groupe VIII non noble, d'au moins un élément promoteur, et de préférence le phosphore, d'au moins une zéolithe Y et d'au moins un liant alumine.

Un catalyseur encore plus préféré comprend, et est de préférence constitué, du nickel, du molybdène, du phosphore, d'une zéolithe USY, et éventuellement aussi une zéolithe béta, et de l'alumine.

Un autre catalyseur préféré comprend, et est de préférence constitué, du nickel, du tungstène, de l'alumine et de la silice-alumine.

Un autre catalyseur préféré comprend, et est de préférence constitué, du nickel, du tungstène, d'une zéolithe USY, de l'alumine et de la silice-alumine.

Ledit catalyseur d'hydrocraquage est par exemple sous forme d'extrudés.

Selon un autre aspect de l'invention, le catalyseur d'hydrocraquage tel que décrit plus haut comprend en outre un ou plusieurs composés organiques contenant de l'oxygène et/ou de l'azote et/ou du soufre. Un tel catalyseur est souvent désigné par le terme "catalyseur additivé". Généralement, le composé organique est choisi parmi un composé comportant une ou plusieurs fonctions chimiques choisies parmi une fonction carboxylique, alcool, thiol, thioéther, sulfone, sulfoxyde, éther, aldéhyde, cétone, ester, carbonate, amine, nitrile, imide, oxime, urée et amide ou encore les composés incluant un cycle furanique ou encore les sucres.

La préparation des catalyseurs des étapes a), b) ou c) est connue et comprend généralement une étape d'imprégnation des métaux du groupe VIII et du groupe VIB lorsqu'il est présent, et éventuellement du phosphore et/ou du bore sur le support, suivie d'un séchage, puis éventuellement d'une calcination. Dans le cas de catalyseur additivé, la préparation se fait généralement par simple séchage sans calcination après introduction du composé organique. On entend ici par calcination un traitement thermique sous un gaz contenant de l'air ou de l'oxygène à une température supérieure ou égale à 200°C. Avant leur utilisation dans une étape du procédé, les catalyseurs sont généralement soumis à une sulfuration afin de former l'espèce active. Le catalyseur de l'étape a) peut aussi être un catalyseur utilisé sous sa forme réduite, impliquant ainsi une étape de réduction dans sa préparation.

Eventuellement, l'étape c) peut mettre en oeuvre une section de chauffe située en amont de la section réactionnelle d'hydrocraquage et dans laquelle l'effluent hydrotraité issu de l'étape b) est chauffé pour atteindre une température adaptée pour d'hydrocraquage, c'est-à-dire une température comprise entre 250 et 480°C. Ladite éventuelle section de chauffe peut ainsi comprendre un ou plusieurs échangeurs, permettant de préférence un échange de chaleur entre l'effluent hydrotraité et l'effluent hydrocraqué, et/ou un four de préchauffe.

**Etape d) de séparation**

Selon l'invention, le procédé de traitement comprend une étape d) de séparation, avantageusement mise en oeuvre dans au moins une section de lavage/séparation, alimentée au moins par l'effluent hydrocraqué issu de l'étape c) et une solution aqueuse, pour obtenir au moins un effluent gazeux, un effluent aqueux et un effluent hydrocarboné.

L'effluent gazeux obtenu à l'issu de l'étape d) comprend avantageusement de l'hydrogène, de préférence comprend au moins 90% volume, de préférence au moins 95% volume, d'hydrogène. Avantageusement, ledit effluent gazeux peut au moins en partie être recyclé vers les étapes a) d'hydrogénation sélective et/ou b) d'hydrotraitement et/ou c) d'hydrocraquage, le système de recyclage pouvant comprendre une section de purification.

L'effluent aqueux obtenu à l'issu de l'étape d) comprend avantageusement des sels d'ammonium et/ou de l'acide chlorhydrique.

L'effluent hydrocarboné issu de l'étape d) comprend des composés hydrocarbonés et correspond avantageusement à l'huile de pyrolyse de plastiques de la charge, ou à l'huile de pyrolyse de plastiques et de la fraction de charge pétrolière ou biomasse conventionnelle co-traitée avec l'huile de pyrolyse, dans laquelle au moins une partie des composés lourds a été convertie en composés plus légers afin de maximiser la coupe naphta. L'effluent hydrocarboné est en plus débarrassé au moins en partie de ses impuretés, en particulier de ses impuretés oléfiniques (di- et mono-oléfines), métalliques, halogénées.

Cette étape d) de séparation permet en particulier d'éliminer les sels de chlorure d'ammonium, qui se forment par réaction entre les ions chlorure, libérés par l'hydrogénation des composés chlorés sous forme HCl notamment lors de l'étape b) puis dissolution dans l'eau, et les ions ammonium, générés par l'hydrogénation des composés azotés sous forme de $NH_3$ notamment lors de l'étape b) et/ou apportés par injection d'une amine puis dissolution dans l'eau, et ainsi de limiter les risques de bouchage, en particulier dans les lignes de transfert et/ou dans les sections du procédé de l'invention et/ou les lignes de transfert vers le vapocraqueur, dû à la précipitation des sels de chlorure d'ammonium. Il permet aussi d'éliminer l'acide chlorhydrique formé par la réaction des ions hydrogène et des ions chlorures.

En fonction de la teneur en composés chlorés dans la charge initiale à traiter, un flux contenant une amine telle que par exemple la monoéthanolamine, la diéthanolamine et/ou la monodiéthanolamine peut être injecté en amont de l'étape a) d'hydrogénation sélective, entre l'étape a) d'hydrogénation sélective et l'étape b) d'hydrotraitement et/ou entre l'étape c) d'hydrocraquage et l'étape d) de séparation, de préférence en amont de l'étape a) d'hydrogénation sélective, afin d'assurer une quantité suffisante en ions ammonium pour combiner les ions chlorure formés lors de l'étape d'hydrotraitement, permettant ainsi de limiter la formation d'acide chlorhydrique et ainsi de limiter la corrosion en aval de la section de séparation.

Avantageusement, l'étape d) de séparation comprend une injection d'une solution aqueuse, de préférence une injection d'eau, dans l'effluent hydrocraqué issu de l'étape c), en amont de la section de lavage/séparation, de manière à dissoudre au moins en partie des sels de chlorure d'ammonium et/ou de l'acide chlorhydrique et améliorer ainsi l'élimination des impuretés chlorées et réduire les risques de bouchages dus à une accumulation des sels de chlorure d'ammonium.

L'étape d) de séparation est avantageusement opérée à une température comprise entre 50 et 370°C, préférentiellement entre 100 et 340°C, de manière préférée entre 200 et 300°C. Avantageusement, l'étape d) de séparation est opérée à une pression proche de celle mise en oeuvre dans les étapes a) et/ou b) et/ou c), de préférence entre 1,0 et 10,0 MPa, de manière à faciliter le recyclage d'hydrogène.

La section de lavage/séparation de l'étape d) peut au moins en partie être réalisée dans des équipements de lavage et de séparation communs ou distincts, ces équipements étant bien connus (ballons séparateurs pouvant opérés à différentes pressions et températures, pompes, échangeurs de chaleurs, colonnes de lavage, etc.).

Dans un mode de réalisation éventuel de l'invention, pris en complément ou isolément d'autres modes de réalisation de l'invention décrits, l'étape d) de séparation comprend l'injection d'une solution aqueuse dans l'effluent hydrocraqué issu de l'étape c), suivi de la section de lavage/séparation comprenant avantageusement une phase de séparation permettant d'obtenir au moins un effluent aqueux chargé en sels d'ammonium, un effluent hydrocarboné liquide lavé et un effluent gazeux partiellement lavé. L'effluent aqueux chargé en sels d'ammonium et l'effluent hydrocarboné liquide lavé peuvent ensuite être séparés dans un ballon décanteur afin d'obtenir ledit effluent hydrocarboné et ledit effluent aqueux. Ledit effluent gazeux partiellement lavé peut parallèlement être introduit dans une colonne de lavage où il circule à contrecourant d'un flux aqueux, de préférence de même nature que la solution aqueuse injectée dans l'effluent hydrocraqué, ce qui permet d'éliminer au moins en partie, de préférence en totalité, l'acide chlorhydrique contenu dans l' effluent gazeux partiellement lavé et d'obtenir ainsi ledit effluent gazeux, comprenant de préférence essentiellement de l'hydrogène, et un flux aqueux acide. Ledit effluent aqueux issu du ballon décanteur peut éventuellement être mélangé avec ledit flux aqueux acide, et être utilisé, éventuellement en mélange avec ledit flux aqueux acide dans un circuit de recyclage d'eau pour alimenter l'étape d) de séparation en ladite solution aqueuse en amont de la section de lavage/séparation et/ou en ledit flux aqueux dans la colonne de lavage. Ledit circuit de recyclage d'eau peut comporter un appoint d'eau et/ou d'une solution basique et/ou une purge permettant d'évacuer les sels dissous.

Dans un autre mode de réalisation éventuel de l'invention, pris séparément ou en combinaison d'autres modes de réalisation de l'invention décrits, l'étape d) de séparation peut comprendre avantageusement une section de lavage/séparation à « haute pression » qui opère à une pression proche de la pression de l'étape a) d'hydrogénation sélective et/ou de l'étape b) d'hydrotraitement et/ou de l'étape c) d'hydrocraquage, afin de faciliter le recyclage d'hydrogène. Cette éventuelle section « haute pression » de l'étape d) peut être complétée par une section « basse pression », afin d'obtenir une fraction liquide hydrocarbonée dépourvue d'une partie des gaz dissous à haute pression et destinée à être traitée directement dans un procédé de vapocraquage ou optionnellement être envoyée dans l'étape e) de fractionnement.

La ou les fractions gaz issue(s) de l'étape d) de séparation peut (peuvent) faire l'objet de purification(s) et de séparation(s) complémentaire(s) en vue de récupérer au moins un gaz riche en hydrogène pouvant être recyclé en amont des étapes a) et/ou b) et/ou c) et/ou des hydrocarbures légers, notamment de l'éthane, du propane et du butane, qui peuvent avantageusement être envoyés séparément ou en mélange dans un ou des fours de l'étape h) de vapocraquage de manière à accroitre le rendement global en oléfines.

L'effluent hydrocarboné issu de l'étape d) de séparation est envoyé, en partie ou en totalité, soit directement en entrée d'une unité de vapocraquage, soit vers une étape e) optionnelle de fractionnement. De préférence, l'effluent liquide hydrocarboné est envoyé, en partie ou en totalité, de manière préférée en totalité, vers une étape e) de fractionnement.

**Etape e) (optionnelle) de fractionnement**

Le procédé selon l'invention peut comprendre une étape de fractionnement de tout ou partie, de manière préférée de la totalité, de l'effluent hydrocarboné issu de l'étape d), pour obtenir au moins un flux gazeux et au moins deux flux hydrocarbonés liquides, lesdits deux flux hydrocarbonés liquides étant au moins une coupe naphta comprenant des composés ayant un point d'ébullition inférieur ou égal à 175°C, en particulier entre 80 et 175°C, et une coupe hydrocarbonée comprenant des composés ayant un point d'ébullition supérieur à 175°C.

L'étape e) permet en particulier d'éliminer les gaz dissous dans l'effluent liquide hydrocarboné, comme par exemple de l'ammoniac, de l'hydrogène sulfuré et des hydrocarbures légers ayant 1 à 4 atomes de carbone.

L'étape e) optionnelle de fractionnement est avantageusement opérée à une pression inférieure ou égale à 1,0 MPa abs., de préférence entre 0,1 et 1,0 MPa abs.

Selon un mode de réalisation, l'étape e) peut être opérée dans une section comprenant avantageusement au moins une colonne de stripage équipée d'un circuit de reflux comprenant un ballon de reflux. Ladite colonne de stripage est alimentée par l'effluent liquide hydrocarboné issu de l'étape d) et par un flux de vapeur d'eau. L'effluent liquide hydrocarboné issu de l'étape d) peut être éventuellement réchauffé avant l'entrée dans la colonne de stripage. Ainsi, les composés les plus légers sont entraînés en tête de colonne et dans le circuit de reflux comprenant un ballon de reflux dans lequel s'opère une séparation gaz/liquide. La phase gazeuse qui comprend les hydrocarbures légers, est soutirée du ballon de reflux, en un flux gazeux. La coupe naphta comprenant des composés ayant un point d'ébullition inférieur ou égal à 175°C est avantageusement soutirée du ballon de reflux. La coupe hydrocarbonée comprenant des composés ayant un point d'ébullition supérieur à 175°C est avantageusement soutirée en fond de colonne de stripage.

Selon d'autres modes de réalisation, l'étape e) de fractionnement peut mettre en oeuvre une colonne de stripage suivie d'une colonne de distillation ou uniquement une colonne de distillation.

La coupe naphta comprenant des composés ayant un point d'ébullition inférieur ou égal à 175°C et la coupe comprenant des composés ayant un point d'ébullition supérieur à 175°C, éventuellement mélangés, peu(ven)t être envoyés, en tout ou partie, vers une unité de vapocraquage, à l'issue de laquelle des oléfines pourront être (re)formées pour participer à la formation de polymères. De préférence, une partie seulement des dites coupes est envoyée vers une unité de vapocraquage ; au moins une fraction de la partie restante est éventuellement envoyée vers l'étape f) ou g) de recyclage et/ou vers un pool carburant, par exemple pool naphta, pool diesel ou pool kérosène, issu de charges pétrolières conventionnelles.

Selon un mode préféré, la coupe naphta comprenant des composés ayant un point d'ébullition inférieur ou égal à 175°C, tout ou partie, est envoyée vers une unité de vapocraquage, tandis que la coupe comprenant des composés ayant un point d'ébullition supérieur à 175°C est envoyée vers l'étape f) de recyclage et/ou vers un pool carburant.

Dans un mode de réalisation particulier, l'étape optionnelle e) de fractionnement peut permettre d'obtenir, outre un flux gazeux, une coupe naphta comprenant des composés ayant un point d'ébullition inférieur ou égal à 175°C, de préférence entre 80 et 175°C, et, une coupe distillats moyens comprenant des composés ayant un point d'ébullition supérieur à 175°C et inférieur à 385°C, et une coupe hydrocarbonée comprenant des composés ayant un point d'ébullition supérieur ou égal à 385°C, dite coupe hydrocarbonée lourde. La coupe naphta peut être envoyée, en tout ou partie, vers une unité de vapocraquage et/ou vers le pool naphta issu de charges pétrolières conventionnelles, elle peut encore être envoyée vers l'étape g) de recyclage; la coupe diesel peut également être, en tout ou partie, soit envoyée vers une unité de vapocraquage, soit vers un pool diesel issu de charges pétrolières conventionnelles, soit envoyée vers l'étape f) de recyclage ; la coupe lourde peut quant à elle être envoyée, au moins en partie, vers une unité de vapocraquage, ou être envoyée vers l'étape f) de recyclage.

Dans un autre mode de réalisation particulier, l'étape optionnelle e) de fractionnement peut permettre d'obtenir, outre un flux gazeux, une coupe naphta comprenant des composés ayant un point d'ébullition inférieur ou égal à 175°C, de préférence entre 80 et 175°C, et une coupe kérosène comprenant des composés ayant un point d'ébullition supérieur à 175°C et inférieur ou égale à 280°C, une coupe diesel comprenant des composés ayant un point d'ébullition supérieur à 280°C et inférieur à 385°C et une coupe hydrocarbonée comprenant des composés ayant un point d'ébullition supérieur ou égal à 385°C, dite coupe hydrocarbonée lourde. La coupe naphta peut être envoyée, en tout ou partie, vers une unité de vapocraquage et/ou vers le pool naphta issu de charges pétrolières conventionnelles, elle peut encore être envoyée vers l'étape g) de recyclage; la coupe kérosène et/ou la coupe diesel peuvent également être, en tout ou partie, soit envoyée vers une unité de vapocraquage, soit respectivement vers un pool kérosène ou diesel issu de charges pétrolières conventionnelles, soit envoyée vers l'étape f) de recyclage ; la coupe lourde peut quant à elle être envoyée, au moins en partie, vers une unité de vapocraquage, ou être envoyée vers l'étape f) de recyclage.

Dans un autre mode de réalisation particulier la coupe naphta comprenant des composés ayant un point d'ébullition inférieur ou égal à 175°C issue de l'étape e) est fractionnée en une coupe naphta lourde comprenant des composés ayant un point d'ébullition entre 80 et 175°C et une coupe naphta légère comprenant des composés ayant un point d'ébullition inférieure à 80°C, au moins une partie de ladite coupe lourde étant envoyée vers un complexe aromatique comportant au moins une étape de reformage du naphta en vue de produire des composés aromatiques. Selon ce mode de réalisation, au moins une partie de_la coupe naphta légère est envoyée dans l'étape h) de vapocraquage décrite ci-dessous.

La ou les fractions gaz issue(s) de l'étape e) de fractionnement peut (peuvent) faire l'objet de purification(s) et de séparation(s) complémentaire(s) en vue de récupérer au moins des hydrocarbures légers, notamment de l'éthane, du propane et du butane, qui peuvent avantageusement être envoyés séparément ou en mélange dans un ou des fours de l'étape h) de vapocraquage de manière à accroître le rendement global en oléfines.

**Etape f) (éventuelle) de recyclage de la coupe comprenant des composés ayant un point d'ébullition supérieur à 175°C**

Le procédé selon l'invention peut comprendre l'étape f) de recyclage, dans laquelle au moins une fraction de la coupe comprenant des composés ayant un point d'ébullition supérieur à 175°C issue de l'étape e) de fractionnement, est récupérée pour constituer un flux de recycle qui est envoyé en amont de ou directement vers au moins l'une des étapes réactionnelles du procédé selon l'invention, en particulier vers l'étape a) d'hydrogénation sélective et/ou l'étape b) d'hydrotraitement et/ou l'étape c) d'hydrocraquage. Eventuellement, une fraction du flux de recycle peut être envoyée vers l'étape a0) optionnelle.

Le flux de recycle peut alimenter lesdites étapes réactionnelles a) et/ou b) et/ou c) en une seule injection ou peut être divisé en plusieurs fractions pour alimenter les étapes réactionnelles a) et/ou b) et/ou c) en plusieurs injections, c'est-à-dire au niveau de différents lits catalytiques.

Avantageusement, la quantité du flux de recycle de la coupe comprenant des composés ayant un point d'ébullition supérieur à 175°C est ajustée de sorte que le rapport pondéral entre le flux de recycle et la charge comprenant une huile de pyrolyse de plastiques, c'est-à-dire la charge à traiter alimentant le procédé global, est inférieur ou égal à 10, de préférence inférieur ou égal à 5, et préférentiellement supérieur ou égal à 0,001, de préférence supérieur ou égal à 0,01, et de manière préférée supérieur ou égal à 0,1. De manière très préférée, la quantité du flux de recycle est ajustée de sorte que le rapport pondéral entre le flux de recycle et la charge comprenant une huile de pyrolyse de plastiques est compris entre 0,2 et 5.

De préférence, le procédé selon l'invention comprend l'étape f) de recyclage. De manière très préférée, au moins une fraction de la coupe comprenant des composés ayant un point d'ébullition supérieur à 175°C issue de l'étape e) de fractionnement est envoyée dans l'étape c) d'hydrocraquage. Le recyclage d'une partie de la coupe comprenant des composés ayant un point d'ébullition supérieur à 175°C vers ou en amont de au moins une des étapes réactionnelles du procédé selon l'invention, et notamment vers l'étape c) d'hydrocraquage permet avantageusement d'augmenter le rendement en coupe naphta ayant un point d'ébullition inférieur à 175°C. Le recyclage permet également de diluer les impuretés et d'autre part de contrôler la température dans la ou les étape(s) réactionnelle(s), dans la(les)quelle(s) des réactions mises en jeu peuvent être fortement exothermiques.

Une purge peut être installée sur le recycle de ladite de la coupe comprenant des composés ayant un point d'ébullition supérieur à 175°C. En fonction des conditions opératoires du procédé, ladite purge peut être comprise entre 0 et 10% poids de la coupe comprenant des composés ayant un point d'ébullition supérieur à 175°C par rapport à la charge entrante, et de préférence entre 0,5% et 5%poids.

**Etape g) (éventuelle) de recyclage de l'effluent hydrocarboné issu de l'étape d) et/ou de la coupe naphta ayant un point d'ébullition inférieur ou égal à 175°C issue de l'étape e)**

Le procédé selon l'invention peut comprendre l'étape g) de recyclage, dans laquelle une fraction de l'effluent hydrocarboné issu de l'étape d) de séparation ou une fraction de la coupe naphta ayant un point d'ébullition inférieur ou égal à 175°C issue de l'étape e) optionnelle de fractionnement, est récupérée pour constituer un flux de recycle qui est envoyé en amont de ou directement vers au moins l'une des étapes réactionnelles du procédé selon l'invention, en particulier vers l'étape a) d'hydrogénation sélective et/ou l'étape b) d'hydrotraitement. Eventuellement, une fraction du flux de recycle peut être envoyée vers l'étape a0) optionnelle de prétraitement. De préférence, le procédé selon l'invention comprend l'étape g) de recyclage.

De préférence, au moins une fraction de l'effluent hydrocarboné issu de l'étape d) de séparation ou de la coupe naphta ayant un point d'ébullition inférieur ou égal à 175°C issue de l'étape e) optionnelle de fractionnement alimente l'étape b) d'hydrotraitement.

Avantageusement, la quantité du flux de recycle, c'est-à-dire la fraction de produit obtenu recyclée, est ajustée de sorte que le rapport pondéral entre le flux de recycle et la charge comprenant une huile de pyrolyse de plastiques, c'est-à-dire la charge à traiter alimentant le procédé global, est inférieur ou égal à 10, de préférence inférieur ou égal à 5, et préférentiellement supérieur ou égal à 0,001, de préférence supérieur ou égal à 0,01, et de manière préférée supérieur ou égal à 0,1. De manière très préférée, la quantité du flux de recycle est ajustée de sorte que le rapport pondéral entre le flux de recycle et la charge comprenant une huile de pyrolyse de plastiques est compris entre 0,2 et 5.

Avantageusement, pour les phases de démarrage du procédé, une coupe hydrocarbonée externe au procédé peut être utilisée comme flux de recycle. L'Homme du métier saura alors choisir ladite coupe hydrocarbonée.

Le recyclage d'une partie du produit obtenu vers ou en amont de au moins une des étapes réactionnelles du procédé selon l'invention permet avantageusement d'une part de diluer les impuretés et d'autre part de contrôler la température dans la ou les étape(s) réactionnelle(s), dans la(les)quelle(s) des réactions mises en jeu peuvent être fortement exothermiques.

Selon un mode de réalisation préféré de l'invention, le procédé de traitement d'une charge comprenant une huile de pyrolyse de plastiques comprend, de préférence consiste en, l'enchainement des étapes, et de préférence dans l'ordre donné, a) d'hydrogénation sélective, b) d'hydrotraitement, c) d'hydrocraquage, d) de séparation et e) de fractionnement, pour produire un effluent dont au moins une partie est compatible pour un traitement dans une unité de vapocraquage.

Selon un autre mode de réalisation préféré de l'invention, le procédé de traitement d'une charge comprenant une huile de pyrolyse de plastiques comprend, de préférence consiste en, l'enchainement des étapes, et de préférence dans l'ordre donné, a0) de prétraitement, a) d'hydrogénation sélective, b) d'hydrotraitement, c) d'hydrocraquage, d) de séparation et e) de fractionnement, pour produire un effluent dont au moins une partie est compatible pour un traitement dans une unité de vapocraquage.

Selon un troisième mode de réalisation préféré de l'invention, le procédé de traitement d'une charge comprenant une huile de pyrolyse de plastiques comprend, de préférence consiste en, l'enchainement des étapes, et de préférence dans l'ordre donné, a) d'hydrogénation sélective, b) d'hydrotraitement, c) d'hydrocraquage, d) de séparation, e) de fractionnement et f) de recyclage de la coupe comprenant des composés ayant un point d'ébullition supérieur à 175°C, pour produire un effluent dont au moins une partie est compatible pour un traitement dans une unité de vapocraquage.

Selon un quatrième mode de réalisation préféré de l'invention, le procédé de traitement d'une charge comprenant une huile de pyrolyse de plastiques comprend, de préférence consiste en, l'enchainement des étapes, et de préférence dans l'ordre donné, a0) de prétraitement, a) d'hydrogénation sélective, b) d'hydrotraitement, c) d'hydrocraquage, d) de séparation, e) de fractionnement et f) de recyclage de la coupe comprenant des composés ayant un point d'ébullition supérieur à 175°C, pour produire un effluent dont au moins une partie est compatible pour un traitement dans une unité de vapocraquage.

Selon un cinquième mode de réalisation préféré de l'invention, le procédé de traitement d'une charge comprenant une huile de pyrolyse de plastiques comprend, de préférence consiste en, l'enchainement des étapes, et de préférence dans l'ordre donné, a) d'hydrogénation sélective, b) d'hydrotraitement, c) d'hydrocraquage, d) de séparation, e) de fractionnement, f) de recyclage de la coupe comprenant des composés ayant un point d'ébullition supérieur à 175°C, g) de recyclage de la coupe comprenant des composés ayant un point d'ébullition inférieur ou égal à 175°C, pour produire un effluent dont au moins une partie est compatible pour un traitement dans une unité de vapocraquage.

Selon un sixième mode de réalisation préféré de l'invention, le procédé de traitement d'une charge comprenant une huile de pyrolyse de plastiques comprend, de préférence consiste en, l'enchainement des étapes, et de préférence dans l'ordre donné, a0) de prétraitement, a) d'hydrogénation sélective, b) d'hydrotraitement, c) d'hydrocraquage, d) de séparation, e) de fractionnement, f) de recyclage de la coupe comprenant des composés ayant un point d'ébullition supérieur à 175°C, g) de recyclage de la coupe comprenant des composés ayant un point d'ébullition inférieur ou égal à 175°C, pour produire un effluent dont au moins une partie est compatible pour un traitement dans une unité de vapocraquage.

Selon un septième mode de réalisation très préféré de l'invention, le procédé de traitement d'une charge comprenant une huile de pyrolyse de plastiques comprend, de préférence consiste en, l'enchainement des étapes, et de préférence dans l'ordre donné, a0) de prétraitement, a) d'hydrogénation sélective, b) d'hydrotraitement, c) d'hydrocraquage, d) de séparation, e) de fractionnement, f) de recyclage de la coupe comprenant des composés ayant un point d'ébullition supérieur à 175°C dans l'étape c), g) de recyclage de la coupe comprenant des composés ayant un point d'ébullition inférieur ou égal à 175°C aux étapes a) et/ou b), pour produire un effluent dont au moins une partie est compatible pour un traitement dans une unité de vapocraquage.

Ledit effluent hydrocarboné ou le(s)dit(s) flux hydrocarboné(s) ainsi obtenu(s) par traitement selon le procédé de l'invention d'une huile de pyrolyse de plastiques, présente(nt) une composition compatible avec les spécifications d'une charge en entrée d'une unité de vapocraquage. En particulier, la composition de l'effluent hydrocarboné ou du(des)dit(s) flux hydrocarboné(s) est de préférence telle que :

- la teneur totale en éléments métalliques est inférieure ou égale à 5,0 ppm poids, de préférence inférieure ou égale à 2,0 ppm poids, préférentiellement inférieure ou égale à 1,0 ppm poids et de manière préférée inférieure ou égale à 0,5 ppm poids, avec :

  une teneur en élément silicium (Si) inférieure ou égale à 1,0 ppm poids, de préférence inférieure ou égale à 0,6 ppm poids, et

  une teneur en élément fer (Fe) inférieure ou égale à 100 ppb poids,

- la teneur en soufre est inférieure ou égale à 500 ppm poids, de préférence inférieure ou égale à 200 ppm poids,

- la teneur en azote est inférieure ou égale à 100 ppm poids, de préférence inférieure ou égale à 50 ppm poids et de manière préférée inférieure ou égale à 5 ppm poids

- la teneur en asphaltènes est inférieure ou égale à 5,0 ppm poids,

- la teneur totale en élément chlore est inférieure ou égale à 10 ppm poids, de manière préférée inférieure à 1,0 ppm poids,

- la teneur en composés oléfiniques (mono- et di-oléfines) est inférieure ou égale à 5,0% poids, de préférence inférieure ou égale à 2,0% poids, de manière préférée inférieure ou égale à 0,1% poids.

Les teneurs sont données en concentrations pondérales relatives, pourcentage (%) poids, partie(s) par million (ppm) poids ou partie(s) par milliard (ppb) poids, par rapport au poids total du flux considéré.

Le procédé selon l'invention permet donc de traiter les huiles de pyrolyse de plastiques pour obtenir un effluent qui peut être injecté, en tout ou partie, dans une unité de vapocraquage.

**Etape h) de vapocraquage (optionnelle)**

L'effluent hydrocarboné issu de l'étape d) de séparation, ou au moins l'un des deux flux hydrocarboné(s) liquides issu(s) de l'étape e) optionnelle, peut être en tout ou partie envoyé vers une étape h) de vapocraquage.

De manière avantageuse, la ou les fractions gaz issue(s) de l'étape d) de séparation et/ou e) de fractionnelle et contenant de l'éthane, du propane et du butane, peut (peuvent) en tout ou partie être également envoyé vers l'étape h) de vapocraquage.

Ladite étape h) de vapocraquage est avantageusement réalisée dans au moins un four de pyrolyse à une température comprise entre 700 et 900°C, de préférence entre 750 et 850°C, et à une pression comprise entre 0,05 et 0,3 MPa relatif. Le temps de séjour des composés hydrocarbonés est généralement inférieur ou égal à 1,0 seconde (noté s), de préférence compris entre 0,1 et 0,5 s. Avantageusement, de la vapeur d'eau est introduite en amont de l'étape h) de vapocraquage optionnelle et après la séparation (ou le fractionnement). La quantité d'eau introduite, avantageusement sous forme de vapeur d'eau, est avantageusement comprise entre 0,3 et 3,0 kg d'eau par kg de composés hydrocarbonés en entrée de l'étape h). De préférence, l'étape h) optionnelle est réalisée dans plusieurs fours de pyrolyse en parallèle de manière à adapter les conditions opératoires aux différents flux alimentant l'étape h) notamment issus de l'étape e), et aussi à gérer les temps de décokage des tubes. Un four comprend un ou plusieurs tubes disposés en parallèle. Un four peut également désigner un groupe de fours opérant en parallèle. Par exemple, un four peut être dédié au craquage de la coupe naphta comprenant des composés ayant un point d'ébullition inférieur ou égal à 175°C.

Les effluents des différents fours de vapocraquage sont généralement recombinés avant séparation en vue de constituer un effluent. Il est entendu que l'étape h) de vapocraquage comporte les fours de vapocraquage mais aussi les sous étapes associées au vapocraquage bien connues de l'Homme du métier. Ces sous étapes peuvent comporter notamment des échangeurs de chaleur, des colonnes et des réacteurs catalytiques et des recyclages vers les fours. Une colonne permet généralement de fractionner l'effluent en vue de récupérer au moins une fraction légère comprenant de l'hydrogène et des composés ayant 2 à 5 atomes de carbone, et une fraction comprenant de l'essence de pyrolyse, et éventuellement une fraction comprenant de l'huile de pyrolyse. Des colonnes permettent de séparer les différents constituants de la fraction légère de fractionnement afin de récupérer au moins une coupe riche en éthylène (coupe C2) et une coupe riche en propylène (coupe C3) et éventuellement une coupe riche en butènes (coupe C4). Les réacteurs catalytiques permettent notamment de réaliser des hydrogénations sélectives des coupes C2, C3 voire C4 et de l'essence de pyrolyse. Les composés saturés, notamment les composés saturés ayant 2 à 4 atomes de carbone sont avantageusement recyclés vers les fours de vapocraquage de manière à accroitre les rendements globaux en oléfines.

Cette étape h) de vapocraquage permet d'obtenir au moins un effluent contenant des oléfines comprenant 2, 3 et/ou 4 atomes de carbone (c'est-à-dire des oléfines en C2, C3 et/ou C4), à des teneurs satisfaisantes, en particulier supérieures ou égales à 30% poids, notamment supérieures ou égales 40% poids, voire supérieures ou égales 50% poids d'oléfines totales comprenant 2, 3 et 4 atomes de carbone par rapport au poids de l'effluent de vapocraquage considéré. Lesdites oléfines en C2, C3 et C4 peuvent ensuite être avantageusement utilisées comme monomères de polyoléfines.

Selon un ou plusieurs mode(s) de réalisation préféré(s) de l'invention, pris séparément ou combinés entre eux, le procédé de traitement d'une charge comprenant une huile de pyrolyse de plastiques comprend, de préférence consiste en, l'enchainement des étapes décrites ci-dessus, et de préférence dans l'ordre donné, c'est-à-dire : a) d'hydrogénation sélective, b) d'hydrotraitement, c) d'hydrocraquage, d) de séparation et l'étape h) de vapocraquage.

Selon un mode préférée, le procédé de traitement d'une charge comprenant une huile de pyrolyse de plastiques comprend, de préférence consiste en, l'enchainement des étapes décrites ci-dessus, et de préférence dans l'ordre donné, c'est-à-dire a0) de prétraitement, a) d'hydrogénation sélective, b) d'hydrotraitement, c) d'hydrocraquage, d) de séparation, e) de fractionnement, f) de recyclage de la coupe comprenant des composés ayant un point d'ébullition supérieur à 175°C dans l'étape c), g) de recyclage de la coupe naphta comprenant des composés ayant un point d'ébullition inférieur ou égal à 175°C aux étapes a) et/ou b), et l'étape h) de vapocraquage.

Le procédé selon l'invention, lorsqu'il comprend cette étape h) de vapocraquage, permet ainsi d'obtenir à partir d'huiles de pyrolyse de plastiques, par exemple de déchets plastiques, des oléfines pouvant servir de monomères à la synthèse de nouveaux polymères contenus dans les plastiques, à des rendements relativement satisfaisants, sans bouchage ni corrosion des unités.

**Méthodes d'analyse utilisées**

Les méthodes d'analyses et/ou normes utilisées pour déterminer les caractéristiques des différents flux en particuliers de la charge à traiter et des effluents, sont connues de l'Homme du métier. Elles sont en particulier listées ci-dessous :

Tableau 1

| Caractéristiques | Méthodes |
|---|---|
| Masse volumique @15°C | ASTM D4052 |
| Teneur en soufre | ISO 20846 |
| Teneur en azote | ASTM D4629 |
| Indice d'acide | ASTM D664 |
| Teneur en Brome | ASTM D1159 |
| Teneur en Di-Oléfines à partir de l'indice d'Anhydride Maléique | Méthode MAV comme décrite dans l'article : C. López-García et al., Near Infrared Monitoring of Low Conjugated Diolefins Content in Hydrotreated FCC Gasoline Streams, Oil & Gas Science and Technology - Rev. IFP, Vol.62 (2007), No.1, 57-68 |
| Teneur en oxygénés | Combustion + Infra-Rouge |
| Teneur en Paraffines | UOP990-11 |

(suite)

| Caractéristiques | Méthodes |
|---|---|
| *Teneur en Naphthènes* | UOP990-11 |
| *Teneur en Oléfines* | UOP990-11 |
| *Teneur en Aromatiques* | UOP990-11 |
| *Teneur en* Halogènes | ASTM-D7359 |
| Teneur en Asphaltènes | IFP9313 |
| Teneur en Chlorure | ASTM D7536 |
| Teneur en métaux : | |
| P | |
| Fe | |
| Si | ASTM-D5185 |
| Na | |
| B | |
| Distillation Simulée | ASTM D2887 |

## LISTE DES FIGURES

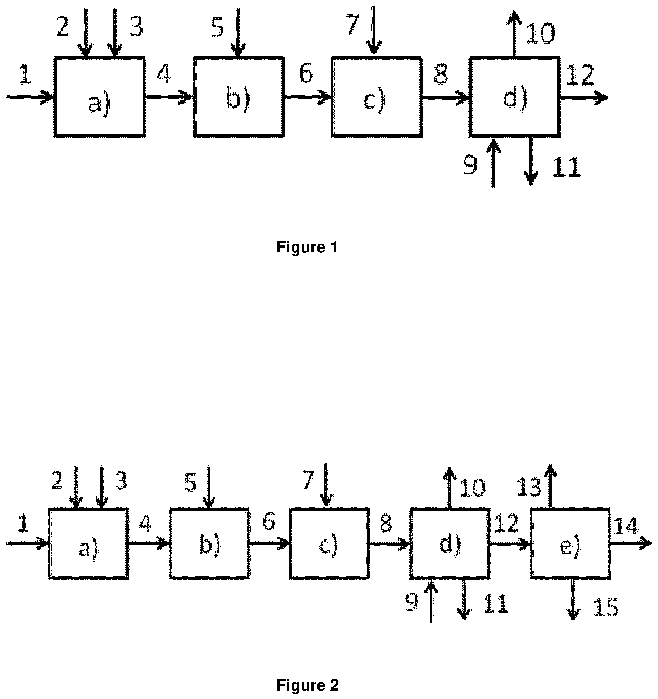

La mention des éléments référencés dans les Figures 1 à 4 permet une meilleure compréhension de l'invention, sans que celle-ci ne se limite aux modes de réalisation particuliers illustrés dans les Figures 1 à 4. Les différents modes de réalisation présentés peuvent être utilisés seul ou en combinaison les uns avec les autres, sans limitation de combinaison.

La Figure 1 représente le schéma d'un mode de réalisation particulier du procédé de la présente invention, comprenant :

- une étape a) d'hydrogénation sélective d'une charge hydrocarbonée issue de la pyrolyse de plastiques 1, en présence d'un gaz riche en hydrogène 2 et éventuellement d'une amine apportée par le flux 3, réalisée dans au moins un réacteur en lit fixe comportant au moins un catalyseur d'hydrogénation sélective, pour obtenir un effluent 4 ;

- une étape b) d'hydrotraitement de l'effluent 4 issu de l'étape a), en présence d'hydrogène 5 réalisée dans au moins un réacteur en lit fixe comportant au moins un catalyseur d'hydrotraitement, pour obtenir un effluent hydrotraité 6 ;

- une étape c) d'hydrocraquage de l'effluent 6 issu de l'étape c), en présence d'hydrogène 7, réalisée dans au moins un réacteur en lit fixe comportant au moins un catalyseur d'hydrocraquage, pour obtenir un effluent hydrocraqué 8 ;

- une étape d) de séparation de l'effluent 8 réalisée en présence d'une solution aqueuse de lavage 9 et permettant d'obtenir au moins une fraction 10 comprenant de l'hydrogène, une fraction aqueuse 11 contenant des sels dissous, et une fraction liquide hydrocarbonée 12.

Au lieu d'injecter le flux d'amine 3 en entrée de l'étape a) d'hydrogénation sélective, il est possible de l'injecter en entrée de l'étape b) d'hydrotraitement, en entrée de l'étape c) d'hydrocraquage, en entrée de l'étape d) de séparation ou encore de ne pas l'injecter, en fonction des caractéristiques de la charge.

La Figure 2 représente un autre mode de réalisation particulier du procédé selon l'invention. Dans le mode de réalisation montré sur la Figure 2, la fraction liquide hydrocarbonée 12, obtenue à l'issue de l'étape d) est envoyée à une étape e) de fractionnement permettant d'obtenir au moins une fraction gazeuse 13, une coupe naphta 14 comprenant des composés ayant un point d'ébullition inférieur ou égal à 175°C et une coupe 15 comprenant des composés ayant un point d'ébullition supérieur à 175°C. A l'issue de l'étape e), la coupe naphta 14 comprenant des composés ayant un point d'ébullition inférieur ou égal à 175°C est envoyée(s) vers un procédé de vapocraquage (non représentée).

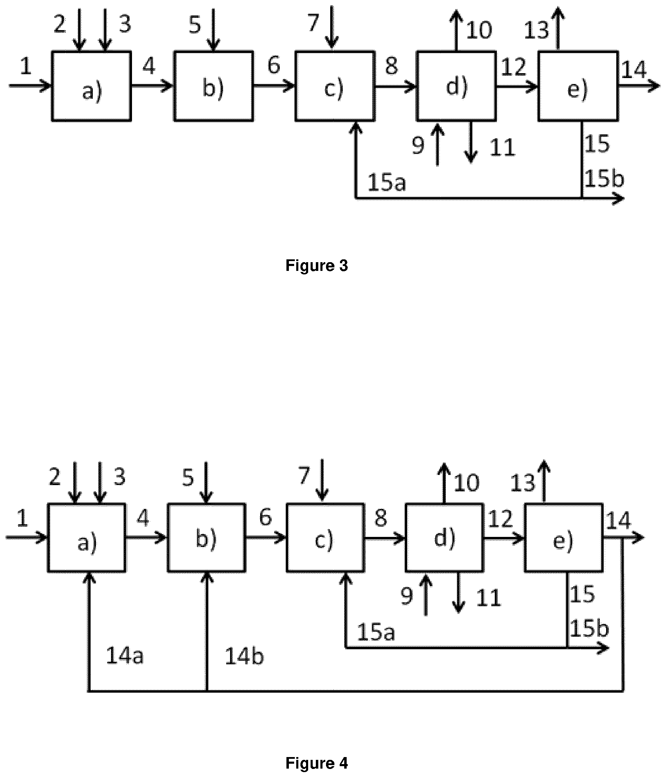

La Figure 3 représente un autre mode de réalisation particulier du procédé selon l'invention. Dans le mode de réalisation montré sur la Figure 3, une partie 15a de la coupe 15 comprenant des composés ayant un point d'ébullition supérieur à 175°C issue de l'étape e)) constitue le flux de recycle qui alimente l'étape c) d'hydrocraquage. Une autre partie 15b constitue la purge.

La Figure 4 représente un autre mode de réalisation particulier du procédé selon l'invention. Dans le mode de réalisation montré sur la Figure 4, une partie de la coupe naphta 14 comprenant des composés ayant un point d'ébullition inférieur ou égal à 175°C issue de l'étape e) constitue le flux de recycle qui alimente l'étape a) d'hydrogénation sélective (fraction 14a), et l'étape b) d'hydrotraitement (fraction 14b).

Seules les principales étapes, avec les flux principaux, sont représentées sur les Figures 1 à 4, afin de permettre une meilleure compréhension de l'invention. Il est bien entendu que tous les équipements nécessaires au fonctionnement sont présents (ballons, pompes, échangeurs, fours, colonnes, etc.), même si non représentés. Il est également entendu que des flux de gaz riche en hydrogène (appoint ou recycle), comme décrit ci-dessus, peuvent être injectés en entrée de chaque réacteur ou lit catalytique ou entre deux réacteurs ou deux lits catalytiques. Des moyens bien connus de l'homme du métier de purification et de recyclage d'hydrogène peuvent être également mis en oeuvre.

## EXEMPLES

### Exemple 1 (conforme à l'invention)

La charge 1 traitée dans le procédé est une huile de pyrolyse de plastiques (c'est-à-dire comprenant 100% poids de ladite huile de pyrolyse de plastiques) présentant les caractéristiques indiquées dans le tableau 2.

Tableau 2 : caractéristiques de la charge

| Description / | Méthodes | Unité | Huile de pyrolyse |
|---|---|---|---|
| Masse volumique @15°C | ASTM D4052 | g/cm$^3$ | 0,820 |
| Teneur en soufre | ISO 20846 | ppm poids | 2500 |
| Teneur en azote | ASTM D4629 | ppm poids | 730 |
| Indice d'acide | ASTM D664 | mgKOH/g | 1,5 |
| Teneur en Brome | ASTM D1159 | g/100g | 80 |
| Teneur en Di-Oléfines à partir de l'Indice d'Anhydride Maléique | Méthode MAV[1] | % poids | 10 |
| Teneur en oxygénés | Combustion + Infra-Rouge | % poids | 1,0 |
| Teneur en Paraffines | UOP990-11 | % poids | 45 |
| Teneur en Naphthènes | UOP990-11 | % poids | 20 |
| Teneur en Oléfines | UOP990-11 | % poids | 25 |
| Teneur en Aromatiques | UOP990-11 | % poids | 10 |
| Teneur en Halogènes | ASTM-D7359 | ppm poids | 350 |
| Teneur en Asphaltènes | IFP9313 | ppm poids | 380 |
| Teneur en Chlorure | ASTM D7536 | ppm poids | 320 |
| Teneur en métaux : | | | |
| P | | ppm poids | 10 |
| Fe | | ppm poids | 25 |
| Si | ASTM-D5185 | ppm poids | 45 |
| Na | | ppm poids | 2 |
| B | | ppm poids | 2 |

(suite)

| Description / | Méthodes | Unité | Huile de pyrolyse |
|---|---|---|---|
| Distillation Simulée : | | | |
| 0% | | °C | 40 |
| 10% | | °C | 98 |
| 30% | ASTM D2887 | °C | 161 |
| 50% | | °C | 232 |
| 70% | | °C | 309 |
| 90% | | °C | 394 |
| 100% | | °C | 432 |
| (1) Méthode MAV décrite dans l'article : C. López-García et al., Near Infrared Monitoring of Low Conjugated Diolefins Content in Hydrotreated FCC Gasoline Streams, Oil & Gas Science and Technology - Rev. IFP, Vol. 62 (2007), No. 1, pp. 57-68 | | | |

La charge 1 est soumise à une étape a) d'hydrogénation sélective réalisée dans un réacteur en lit fixe et en présence d'hydrogène 2 et d'un catalyseur d'hydrogénation sélective de type NiMo sur alumine dans les conditions indiquées dans le tableau 3.

Tableau 3 : conditions de l'étape a) d'hydrogénation sélective

| Température | °C | 180 |
|---|---|---|
| Pression Partielle d'Hydrogène | MPa abs | 6,4 |
| $H_2$/HC (Couverture volumique d'hydrogène par rapport au volume de charge) | $Nm^3/m^3$ | 50 |
| VVH (débit volumique de charge/volume de catalyseurs) | h-1 | 0,5 |

A l'issue de l'étape a) d'hydrogénation sélective, la totalité des dioléfines initialement présentes dans la charge ont été converties.

L'effluent 4 issu de l'étape a) d'hydrogénation sélective est soumis directement, sans séparation, à une étape b) d'hydrotraitement réalisée en lit fixe et en présence d'hydrogène 5, et d'un catalyseur d'hydrotraitement de type NiMo sur alumine dans les conditions présentées dans le tableau 4.

Tableau 4 : conditions de l'étape b) d'hydrotraitement

| Température d'hydrotraitement | °C | 355 |
|---|---|---|
| Pression Partielle d'Hydrogène | MPa abs | 6,2 |
| $H_2$/HC (Couverture volumique d'hydrogène par rapport au volume de charge) | $Nm^3/m^3$ | 300 |
| VVH (débit volumique de charge/volume de catalyseurs) | $h^{-1}$ | 0,5 |

L'effluent 6 issu de l'étape b) d'hydrotraitement est soumis directement, sans séparation, à une étape c) d'hydrocraquage réalisée en lit fixe et en présence d'hydrogène 7 et d'un catalyseur d'hydrocraquage zéolitique comprenant du NiMo dans les conditions présentées dans le tableau 5.

Tableau 5 : conditions de l'étape c) d'hydrocraquage

| Température d'hydrocraquage | °C | 350 |
|---|---|---|
| Pression Partielle d'Hydrogène | MPa abs | 5,8 |
| $H_2$/HC (Couverture volumique d'hydrogène par rapport au volume de charge) | $Nm^3/m^3$ | 350 |

(suite)

| VVH (débit volumique de charge/volume de catalyseurs) | h⁻¹ | 2 |
|---|---|---|

L'effluent 8 issu de l'étape c) d'hydrocraquage est soumis à une étape d) de séparation selon l'invention dans laquelle un flux d'eau 9 est injecté dans l'effluent issu de l'étape c) d'hydrocraquage; le mélange est ensuite envoyé dans l'étape d) de séparation et est traité dans une colonne de lavage des gaz acides. Une fraction gaz 10 est obtenue en tête de la colonne de lavage des gaz acides tandis qu'en fond, un ballon séparateur diphasique permet de séparer une phase aqueuse et une phase liquide. La colonne de lavage de gaz et le séparateur diphasique sont opérés à haute pression. La phase liquide est ensuite envoyée dans un ballon basse pression de manière à récupérer une seconde fraction gazeuse qui est purgée et un effluent liquide. Les rendements des différents produits et des différentes fractions obtenues en sortie de l'étape c) d'hydrocraquage sont indiqués dans le tableau 6 (les rendements étant correspondant aux rapports des quantités massiques des différents produits obtenus par rapport à la masse de charge en amont de l'étape a), exprimés en pourcentage et notés % m/m).

Tableau 6 : rendements des différents produits et fractions obtenus en sortie de l'étape c) d'hydrocraquage

| $H_2S$ | % m/m | 0,27 |
|---|---|---|
| $NH_3$ | % m/m | 0,09 |
| C1 | % m/m | 0,17 |
| C2 | % m/m | 0,26 |
| C3 | % m/m | 1,65 |
| C4 | % m/m | 7,01 |
| Fraction PI+ | | 92,69 |
| Total | % m/m | 102,14 |

Les caractéristiques de la fraction PI+ (qui correspond à l'effluent liquide 12) obtenue après l'étape d) de séparation sont présentés tableau 7 :

Tableau 7 : caractéristiques de la fraction PI+

| | | Fraction PI+ |
|---|---|---|
| Masse volumique @ 15°C (ASTM D4052) | g/cm³ | 0,759 |
| Teneur en : | | |
| Soufre (ASTM D5453) | ppm poids | < 2 |
| Azote (ASTM D4629) | ppm poids | < 0,8 |
| Fe (ASTM D5185) | ppb poids | Non détecté |
| Métaux Totaux (ASTM D5185) | ppm poids | Non détecté |
| Chlore (ASTM D7536) | ppb poids | Non détecté |
| Paraffines (UOP990-11) | % poids | 80 |
| Naphthènes (UOP990-11) | % poids | 19 |
| Oléfines (UOP990-11) | % poids | Non détecté |
| Aromatiques (UOP990-11) | % poids | 0,5 |
| Distillation Simulée (ASTM D2887) en % | | |
| 0 | °C | 25 |
| 5 | °C | 40 |
| 10 | °C | 61 |

(suite)

| Distillation Simulée (ASTM D2887) en % | | |
|---|---|---|
| 30 | °C | 94 |
| 50 | °C | 123 |
| 70 | °C | 157 |
| 90 | °C | 179 |
| 95 | °C | 258 |
| 100 | °C | 350 |

La fraction liquide PI+ présente une composition compatible avec une unité de vapocraquage puisque :

- elle ne contient pas d'oléfines (mono- et di-oléfines) ;

- elle ne contient pas de chlore (teneur non détectée et inférieure à la limite requise pour une charge de vapocraqueur

- elle ne contient ni fer (Fe) ni métaux (teneurs en métaux non détectées et inférieures aux limites requises pour une charge de vapocraqueur soit $\leq$ 5,0 ppm poids et de manière très préférée $\leq$ 1 ppm poids pour les métaux ; et aussi $\leq$ 100 ppb poids pour le Fe) ;

- enfin elle contient peu de soufre (< 2 ppm poids) et peu dazote (< 0,8 ppm poids) , ces teneurs sont très inférieures aux limites requises pour une charge de vapocraqueur ($\leq$ 500 ppm poids, de préférence $\leq$ 200 ppm poids pour S et N).

La fraction PI+ obtenue via l'enchainement d'étapes et notamment l'étape c) d'hydrocraquage est constituée d'environ 86% de composés de type naphta ayant un point d'ébullition inférieure ou égal à 175°C. Ce rendement élevé en composés de type naphta ayant un point d'ébullition inférieur ou égal à 175°C est obtenu grâce aux conditions d'hydrocraquage.

L'effluent liquide PI+ est envoyé directement vers une étape h) de vapocraquage selon les conditions mentionnées dans le tableau 8.

Tableau 8 : conditions de l'étape de vapocraquage

| Pression sortie fours | MPa abs | 0,2 |
|---|---|---|
| Température sortie four fractions PI+ | °C | 795 |
| Ratio Vapeur / fractions PI+ | kg/kg | 0,7 |
| Temps de séjour four fractions PI+ | s | 0,3 |

L'effluent du four de vapocraquage est soumis à une étape de séparation permettant de recycler les composés saturés vers le four de vapocraquage et d'obtenir les rendements présentés dans le tableau 9 (rendement = % de masse de produit par rapport à la masse de fraction PI+ en amont de l'étape de vapocraquage, noté % m/m).

Tableau 9 : rendements de l'étape de vapocraquage

| Fractions | | Fraction PI+ |
|---|---|---|
| $H_2$, CO, C1 | % m/m | 7,9 |
| Ethylène | % m/m | 34,1 |
| Propylène | % m/m | 18,6 |
| Coupe C4 | % m/m | 14,8 |
| Essence de pyrolyse | % m/m | 19,5 |
| Huile de pyrolyse | % m/m | 5,2 |

En considérant le rendement obtenu pour la fraction PI+ lors du procédé de traitement de l'huile de pyrolyse (cf. tableau 6), il est possible de déterminer les rendements globaux des produits issus de l'étape de vapocraquage par rapport à la charge initiale de type huile de pyrolyse de plastiques introduite à l'étape a) :

Tableau 10 : rendements globaux du procédé en produits issus de l'étape de vapocraquage

| Fractions | | Fraction PI+ |
|---|---|---|
| $H_2$, CO, C1 | % m/m | 7,3 |
| Ethylène | % m/m | 31,6 |
| Propylène | % m/m | 17,2 |
| Coupe C4 | % m/m | 13,7 |
| Essence de pyrolyse | % m/m | 18,0 |
| Huile de pyrolyse | % m/m | 4,8 |

Lorsque la fraction liquide PI+ est soumise à une étape de vapocraquage, le procédé selon l'invention permet d'atteindre des rendements massiques globaux en éthylène et en propylène respectivement de 31,6 % et 17,2 % par rapport à la quantité massique de charge de type huile de pyrolyse de plastiques initiale.
De plus, l'enchainement spécifique d'étapes en amont de l'étape de vapocraquage permet de limiter la formation de coke et d'éviter les problèmes de corrosion qui seraient apparus si le chlore n'avait pas été éliminé.

**Exemple 2 (conforme à l'invention)**

Dans cet exemple, la charge à traiter est identique à celle décrite dans l'Exemple 1 (cf. tableau 2).
Elle subit les étapes a) d'hydrogénation sélective, b) d'hydrotraitement, c) d'hydrocraquage et d) de séparation, opérées dans les mêmes conditions que celles décrites dans l'Exemple 1. L'effluent liquide obtenu à l'issue de l'étape d) de séparation est envoyée vers une étape e) de fractionnement comprenant une colonne de stripage et une colonne de distillation en vue d'obtenir une fraction ayant un point d'ébullition inférieure ou égal à 175°C (fraction PI-175°C) et une fraction ayant un point d'ébullition supérieure à 175°C (fraction 175°C+).
Le tableau 11 donne les rendements globaux des différentes fractions obtenues à l'issue des étapes d) de séparation et e) de fractionnement (qui comprend une colonne de stripage et une colonne de distillation).

Tableau 11 : rendements des différents produits et fractions obtenus en sortie de l'étape c) d'hydrocraquage

| $H_2S$ | % m/m | 0,27 |
|---|---|---|
| $NH_3$ | % m/m | 0,09 |
| C1 | % m/m | 0,17 |
| C2 | % m/m | 0,26 |
| C3 | % m/m | 1,65 |
| C4 | % m/m | 7,01 |
| Fraction PI-175°C | % m/m | 84,69 |
| Fraction 175°C+ | % m/m | 8,00 |
| Total | % m/m | 102,14 |

Le traitement de la charge selon les étapes de l'invention et notamment l'étape c) d'hydrocraquage permet d'obtenir un rendement très élevé en fraction PI-175°C de type naphta.
Les caractéristiques des fractions liquides PI-175°C et 175°C+ obtenues après l'étape d) de séparation et une étape e) de fractionnement sont présentés tableau 12 :

Tableau 12 : caractéristiques des fraction PI-175°C, 175°C+

| | | Fraction PI-175°C | Fraction 175°C+ |
|---|---|---|---|
| Masse volumique @ 15°C (ASTM D4052) | g/cm$^3$ | 0,755 | 0,810 |
| Teneur en : | | | |
| Soufre (ASTM D5453) | ppm poids | <2 | <2 |
| Azote (ASTM D4629) | ppm poids | < 0,5 | < 3 |
| Fe (ASTM D5185) | ppb poids | Non détecté | 25 |
| Métaux Totaux (ASTM D5185) | ppm poids | Non détecté | 1 |
| Chlore (ASTM D7536) | ppb poids | Non détecté | 25 |
| Paraffines (UOP990-11) | % poids | 80 | 83 |
| Naphthènes (UOP990-11) | % poids | 19,5 | 15 |
| Oléfines (UOP990-11) | % poids | Non détecté | Non détecté |
| Aromatiques (UOP990-11) | % poids | 0,5 | 1 |
| Distillation Simulée (ASTM D2887) en % | | | |
| 0 | °C | 25 | 175 |
| 5 | °C | 38 | 185 |
| 10 | °C | 59 | 206 |
| 30 | °C | 90 | 235 |
| 50 | °C | 118 | 265 |
| 70 | °C | 140 | 285 |
| 90 | °C | 163 | 320 |
| 95 | °C | 175 | 344 |
| 100 | °C | 180 | 350 |

Les fractions liquides PI-175°C et 175°C+ présentent toutes les deux des compositions compatibles avec une unité de vapocraquage puisque :

- elles ne contiennent pas d'oléfines (mono- et di-oléfines) ;

- elles présentent des teneurs en élément chlore très faibles (respectivement une teneur non détectée et une teneur de 25 ppb poids) et inférieures à la limite requise pour une charge de vapocraqueur;

- les teneurs en métaux, en particulier en fer (Fe), sont elles aussi très faibles (teneurs en métaux non détectée pour la fraction PI-175°C et < 1 ppm poids pour la fraction 175°C+ ; teneurs en Fe non détectée pour la fraction PI-175°C et 25 ppb poids pour la fraction 175°C+) et inférieures aux limites requises pour une charge de vapocraqueur ($\leq$ 5,0 ppm poids, de manière très préférée $\leq$ 1 ppm poids pour les métaux ; $\leq$ 100 ppb poids pour le Fe) ;

- enfin elles contiennent du soufre (< 2 ppm poids pour la fraction PI-175°C et < 2 ppm poids pour la fraction 175°C+) et de l'azote (< 0,5 ppm poids pour la fraction PI-175°C et < 3 ppm poids pour la fraction 175°C+) à des teneurs très inférieures aux limites requises pour une charge de vapocraqueur ($\leq$ 500 ppm poids, de préférence $\leq$ 200 ppm poids pour S et N).

Les fractions liquides PI-175°C et 175°C+ obtenues sont donc ensuite envoyées vers une étape h) de vapocraquage où les fractions liquides sont craquées dans des conditions différentes (cf. tableau 13).

Tableau 13 : conditions de l'étape de vapocraquage

| Pression sortie fours | MPa abs | 0,2 |
|---|---|---|
| Température sortie four fractions PI-175°C | °C | 800 |
| Température sortie four fraction 175°C+ | °C | 790 |
| Ratio Vapeur / fractions PI-175°C | kg/kg | 0,6 |
| Ratio Vapeur / fraction 175°C+ | kg/kg | 0,8 |
| Temps de séjour four fractions PI-175°C | s | 0,3 |
| Temps de séjour four fractions 175°C+ | s | 0,3 |

Les effluents des différents fours de vapocraquage sont soumis à une étape de séparation permettant de recycler les composés saturés vers les fours de vapocraquage et d'obtenir les rendements présentés dans le tableau 14 (rendement = % de masse de produit par rapport à la masse de chacune des fractions en amont de l'étape de vapocraquage, noté % m/m).

Tableau 14 : rendements de l'étape de vapocraquage

| Fractions | | Fraction PI-175°C | Fraction 175°C+ |
|---|---|---|---|
| $H_2$, CO, C1 | % m/m | 7,9 | 8,0 |
| Ethylène | % m/m | 34,1 | 34,7 |
| Propylène | % m/m | 18,6 | 18,9 |
| Coupe C4 | % m/m | 14,8 | 15,1 |
| Essence de pyrolyse | % m/m | 19,5 | 18,9 |
| Huile de pyrolyse | % m/m | 5,2 | 4,4 |

En considérant les rendements obtenus pour les différentes fractions liquides PI-175°C et 175°C+ lors du procédé de traitement de l'huile de pyrolyse en sortie de l'étape c) d'hydrocraquage (cf. tableau 11), il est possible de déterminer les rendements globaux des produits issus de l'étape de vapocraquage par rapport à la charge initiale de type huile de pyrolyse de plastiques introduite à l'étape a) :

Tableau 15 : rendements globaux du procédé en produits issus de l'étape de vapocraquage

| Fractions | | Fraction PI-175°C | Fraction 175°C+ |
|---|---|---|---|
| $H_2$, CO, C1 | % m/m | 6,7 | 0,6 |
| Ethylène | % m/m | 28,9 | 2,8 |
| Propylène | % m/m | 15,7 | 1,5 |
| Coupe C4 | % m/m | 12,5 | 1,2 |
| Essence de pyrolyse | % m/m | 16,5 | 1,5 |
| Huile de pyrolyse | % m/m | 4,4 | 0,4 |

Lorsque les fractions PI-175°C et 175°C+ sont envoyées à l'unité de vapocraquage séparément, le procédé selon l'invention permet d'atteindre des rendements massiques globaux en éthylène et en propylène respectivement de 31,7 % (= 28,9 + 2,8) et 17,2 % (= 15,7 + 1,5) par rapport à la quantité massique de charge de type huile de pyrolyse de plastiques initiale.

De plus, l'enchainement spécifique d'étapes en amont de l'étape de vapocraquage permet de limiter la formation de coke et d'éviter les problèmes de corrosion qui seraient apparus si le chlore n'avait pas été éliminé.

**Exemple 3 (conforme à l'invention)**

Dans cet exemple, la charge à traiter est identique à celle décrite dans l'Exemple 1 (cf. tableau 2).

Elle subit les étapes a) d'hydrogénation sélective, b) d'hydrotraitement, c) d'hydrocraquage et d) de séparation, opérées dans les mêmes conditions que celles décrites dans l'Exemple 1. L'effluent liquide obtenu à l'issue de l'étape d) de séparation est envoyée vers une étape e) de fractionnement comprenant une colonne de stripage et une colonne de distillation en vue d'obtenir une fraction PI-175°C et une fraction 175°C+. La fraction 175°C+ est en partie recyclée en amont de l'étape c) d'hydrocraquage de manière à accroitre la conversion des composés ayant un point d'ébullition supérieur à 175°C. Une faible partie de la fraction 175°C n'est pas recyclée en amont de l'étape c) d'hydrocraquage (purge) de manière à éviter l'accumulation de composés polyaromatiques qui pourraient être précurseur de coke.

Le débit volumique de fraction 175°C+ issue de l'étape e) de fractionnement et recyclé en amont de l'étape c) d'hydrocraquage est égal à la moitié du débit volumique de l'effluent liquide issu de l'étape b) d'hydrotraitement.

Le tableau 16 donne les rendements globaux des différentes fractions obtenues à l'issue des étapes c) de séparation et d) de fractionnement (qui comprend une colonne de stripage et une colonne de distillation).

Tableau 16 : rendements des différents produits et fractions obtenus en sortie de l'étape c) d'hydrocraquage

| H$_2$S | % m/m | 0,27 |
|---|---|---|
| NH$_3$ | % m/m | 0,09 |
| C1 | % m/m | 0,19 |
| C2 | % m/m | 0,28 |
| C3 | % m/m | 2,50 |
| C4 | % m/m | 9,30 |
| Fraction PI-175°C | % m/m | 89,40 |
| Fraction 175°C+ | % m/m | 0,50 |
| Total | % m/m | 102,52 |

Le traitement de la charge selon les étapes de l'invention et notamment l'étape c) d'hydrocraquage permet d'obtenir un rendement très élevé en fraction PI-175°C de type naphta, qui est encore accrue par rapport à l'exemple 1 du fait du recyclage partiel mais quasi-total de la fraction 175°C+ en entrée de l'étape c) d'hydrocraquage.

Les caractéristiques des fractions liquides PI-175°C et 175°C+ obtenues après l'étape d) de séparation et une étape e) de fractionnement sont présentés tableau 17 :

Tableau 17 : caractéristiques des fraction PI-175°C, 175°C+

| | | Fraction PI-175°C | Fraction 175°C+ |
|---|---|---|---|
| Masse volumique @ 15°C (ASTM D4052) | g/cm$^3$ | 0.753 | 0.805 |
| Teneur en : | | | |
| Soufre (ASTM D5453) | ppm poids | <2 | <2 |
| Azote (ASTM D4629) | ppm poids | < 0.5 | < 3 |
| Fe (ASTM D5185) | ppb poids | Non détecté | 25 |
| Métaux Totaux (ASTM D5185) | ppm poids | Non détecté | 1 |
| Chlore (ASTM D7536) | ppb poids | Non détecté | < 25 |
| Paraffines (UOP990-11) | % poids | 81.5 | 83 |
| Naphthènes (UOP990-11) | % poids | 18 | 15 |
| Oléfines (UOP990-11) | % poids | Non détecté | 0 |
| Aromatiques (UOP990-11) | % poids | 0.5 | 1 |
| Distillation Simulée (ASTM D2887) en % | | | |
| 0 | °C | 25 | 175 |
| 5 | °C | 38 | 185 |
| 10 | °C | 59 | 206 |

(suite)

| Distillation Simulée (ASTM D2887) en % | | | |
|---|---|---|---|
| 30 | °C | 90 | 235 |
| 50 | °C | 118 | 265 |
| 70 | °C | 140 | 285 |
| 90 | °C | 163 | 320 |
| 95 | °C | 175 | 344 |
| 100 | °C | 180 | 350 |

Les fractions liquides PI-175°C et 175°C+ présentent toutes les deux des compositions compatibles avec une unité de vapocraquage puisque :

- elles ne contiennent pas d'oléfines (mono- et di-oléfines) ;

- elles présentent des teneurs en élément chlore très faibles (respectivement une teneur non détectée et une teneur de 25 ppb poids) et inférieures à la limite requise pour une charge de vapocraqueur ;

- les teneurs en métaux, en particulier en fer (Fe), sont elles aussi très faibles (teneurs en métaux non détectée pour la fraction PI-175°C et < 1 ppm poids pour la fraction 175°C+ ; teneurs en Fe non détectée pour la fraction PI-175°C et 25 ppb poids pour la fraction 175°C+) et inférieures aux limites requises pour une charge de vapocraqueur ($\leq$ 5,0 ppm poids, de manière très préférée $\leq$ 1 ppm poids pour les métaux ; $\leq$ 100 ppb poids pour le Fe) ;

- enfin elles contiennent du soufre (< 2 ppm poids pour la fraction PI-175°C et < 2 ppm poids pour la fraction 175°C+) et de l'azote (< 0,5 ppm poids pour la fraction PI-175°C et < 3 ppm poids pour la fraction 175°C+) à des teneurs très inférieures aux limites requises pour une charge de vapocraqueur ($\leq$ 500 ppm poids, de préférence $\leq$ 200 ppm poids pour S et N).

Dans cet exemple 2, la fraction 175°C+ est purgée de l'unité et n'est pas envoyée à l'étape de vapocraquage.
La fraction liquide PI-175°C obtenue est donc ensuite envoyées vers une étape h) de vapocraquage (cf. tableau 18).

Tableau 18 : conditions de l'étape de vapocraquage

| Pression sortie fours | MPa abs | 0,2 |
|---|---|---|
| Température sortie four fractions PI-175°C | °C | 800 |
| Ratio Vapeur / fractions PI-175°C | kg/kg | 0,6 |
| Temps de séjour four fractions PI-175°C | s | 0,3 |

Les effluents des différents fours de vapocraquage sont soumis à une étape de séparation permettant de recycler les composés saturés vers les fours de vapocraquage et d'obtenir les rendements présentés dans le tableau 19 (rendement = % de masse de produit par rapport à la masse de chacune des fractions en amont de l'étape de vapocraquage, noté % m/m).

Tableau 19 : rendements de l'étape de vapocraquage

| Fractions | | Fraction PI-175°C |
|---|---|---|
| $H_2$, CO, C1 | % m/m | 7,9 |
| Ethylène | % m/m | 34,3 |
| Propylène | % m/m | 18,7 |
| Coupe C4 | % m/m | 14,9 |
| Essence de pyrolyse | % m/m | 19,3 |

(suite)

| Fractions | | Fraction PI-175°C |
|---|---|---|
| Huile de pyrolyse | % m/m | 4,9 |

En considérant les rendements obtenus pour la fraction liquide 175°C+ lors du procédé de traitement de l'huile de pyrolyse en sorti de l'étape d'hydrocraquage (cf. tableau 16), il est possible de déterminer les rendements globaux des produits issus de l'étape de vapocraquage par rapport à la charge initiale de type huile de pyrolyse de plastiques introduite à l'étape a) :

Tableau 20 : rendements globaux du procédé en produits issus de l'étape de vapocraquage

| Fractions | | Fraction PI-175°C |
|---|---|---|
| $H_2$, CO, C1 | % m/m | 7,1 |
| Ethylène | % m/m | 30,7 |
| Propylène | % m/m | 16,7 |
| Coupe C4 | % m/m | 13,3 |
| Essence de pyrolyse | % m/m | 17,2 |
| Huile de pyrolyse | % m/m | 4,4 |

Lorsque la fraction PI-175°C est envoyée à l'unité de vapocraquage séparément, le procédé selon l'invention permet d'atteindre des rendements massiques globaux en éthylène et en propylène respectivement de 30,7 % et 16,7 % par rapport à la quantité massique de charge de type huile de pyrolyse de plastiques initiale.

De plus, l'enchainement spécifique d'étapes en amont de l'étape de vapocraquage permet de limiter la formation de coke et d'éviter les problèmes de corrosion qui seraient apparus si le chlore n'avait pas été éliminé.

**Exemple 4 (non conforme à l'invention)**

Dans cet exemple, la charge à traiter est identique à celle décrite dans l'Exemple 1 (cf. tableau 2).

Elle subit les étapes a) d'hydrogénation sélective, b) d'hydrotraitement et d) de séparation, opérées dans les mêmes conditions que celles décrites dans l'Exemple 1. Dans cet exemple non conforme à l'invention, l'effluent issu de l'étape d'hydrotraitement n'est pas soumis à une étape d'hydrocraquage. L'effluent liquide obtenu à l'issue de l'étape d) de séparation constitue la fraction PI+.

Les rendements des différents produits et des différentes fractions obtenues en sortie de l'étape b) d'hydrotraitement sont indiqués dans le tableau 21 (les rendements étant correspondant aux rapports des quantités massiques des différents produits obtenus par rapport à la masse de charge en amont de l'étape a), exprimés en pourcentage et notés % m/m).

Tableau 21 : rendements des différents produits et fractions obtenus en sortie de l'étape b) d'hydrotraitement

| $H_2S$ | % m/m | 0,27 |
|---|---|---|
| $NH_3$ | % m/m | 0,09 |
| C1 | % m/m | 0,01 |
| C2 | % m/m | 0,02 |
| C3 | % m/m | 0,09 |
| C4 | % m/m | 0,38 |
| Fraction PI+ | | 99,55 |
| Total | % m/m | 100,05 |

Les caractéristiques de la fraction PI+ (qui correspond à l'effluent liquide) obtenue après l'étape d) de séparation sont présentés tableau 22 :

Tableau 22 : caractéristiques de la fraction PI+

| | | Fraction PI+ |
|---|---|---|
| Masse volumique @ 15°C (ASTM D4052) | g/cm$^3$ | 0,803 |
| Teneur en : | | |
| Soufre (ASTM D5453) | ppm poids | < 2 |
| Azote (ASTM D4629) | ppm poids | < 0,8 |
| Fe (ASTM D5185) | ppb poids | Non détecté |
| Métaux Totaux (ASTM D5185) | ppm poids | Non détecté |
| Chlore (ASTM D7536) | ppb poids | Non détecté |
| Paraffines (UOP990-11) | % poids | 36 |
| Naphthènes (UOP990-11) | % poids | 32 |
| Oléfines (UOP990-11) | % poids | Non détecté |
| Aromatiques (UOP990-11) | % poids | 2 |
| Distillation Simulée (ASTM D2887) en % | | |
| 0 | °C | 26 |
| 5 | °C | 55 |
| 10 | °C | 93 |
| 30 | °C | 157 |
| 50 | °C | 226 |
| 70 | °C | 304 |
| 90 | °C | 390 |
| 95 | °C | 397 |
| 100 | °C | 431 |

La fraction PI+ obtenue via l'enchainement d'étapes a), b) et d) est constituée d'environ 35% de composés de type naphta ayant un point d'ébullition inférieure ou égale à 175°C. Ce rendement faible en composés de type naphta ayant un point d'ébullition inférieur ou égal à 175°C est dû à l'absence d'étape d'hydrocraquage dans cet exemple non conforme.

L'effluent liquide fraction PI+ est envoyé directement vers une étape h) de vapocraquage selon les conditions mentionnées dans le tableau 23.

Tableau 23 : conditions de l'étape de vapocraquage

| | | |
|---|---|---|
| Pression sortie fours | MPa abs | 0,2 |
| Température sortie four fraction PI+ | °C | 795 |
| Ratio Vapeur / fraction PI+ | kg/kg | 0,7 |
| Temps de séjour four fraction PI+ | s | 0,3 |

L'effluent du four de vapocraquage est soumis à une étape de séparation permettant de recycler les composés saturés vers le four de vapocraquage et d'obtenir les rendements présentés dans le tableau 24 (rendement = % de masse de produit par rapport à la masse de fraction PI+ en amont de l'étape de vapocraquage, noté % m/m).

Tableau 24 : rendements de l'étape de vapocraquage

| Fractions | | Fraction PI+ |
|---|---|---|
| H$_2$, CO, C1 | % m/m | 8,1 |
| Ethylène | % m/m | 34,8 |
| Propylène | % m/m | 19,0 |
| Coupe C4 | % m/m | 15,1 |
| Essence de pyrolyse | % m/m | 18,8 |
| Huile de pyrolyse | % m/m | 4,2 |

En considérant le rendement obtenu pour la fraction PI+ lors du procédé de traitement de l'huile de pyrolyse en sortie de l'étape b) d'hydrotraitement (cf. tableau 21), il est possible de déterminer les rendements globaux des produits issus de l'étape de vapocraquage par rapport à la charge initiale de type huile de pyrolyse de plastiques introduite à l'étape a) :

Tableau 25 : rendements globaux du procédé en produits issus de l'étape de vapocraquage

| Fractions | | Fraction PI+ |
|---|---|---|
| H$_2$, CO, C1 | % m/m | 8,1 |
| Ethylène | % m/m | 34,6 |
| Propylène | % m/m | 18,9 |
| Coupe C4 | % m/m | 15,0 |
| Essence de pyrolyse | % m/m | 18,7 |
| Huile de pyrolyse | % m/m | 4,2 |

Lorsque la fraction liquide PI+ est soumise à une étape de vapocraquage, le procédé selon l'invention permet d'atteindre des rendements massiques globaux en éthylène et en propylène respectivement de 34,6 % et 18,9 % par rapport à la quantité massique de charge de type huile de pyrolyse de plastiques initiale.

**Revendications**

1. Procédé de traitement d'une charge comprenant une huile de pyrolyse de plastiques comprenant des composés chlorés, comprenant :

a) une étape d'hydrogénation sélective mise en oeuvre dans une section réactionnelle alimentée au moins par ladite charge et un flux gazeux comprenant de l'hydrogène, en présence d'au moins un catalyseur d'hydrogénation sélective, à une température entre 100 et 280°C, une pression partielle d'hydrogène entre 1,0 et 10,0 MPa abs. et une vitesse volumique horaire entre 0,3 et 10,0 h$^{-1}$, pour obtenir un effluent hydrogéné ;
b) une étape d'hydrotraitement mise en oeuvre dans une section réactionnelle d'hydrotraitement, mettant en oeuvre au moins un réacteur à lit fixe ayant n lits catalytiques, n étant un nombre entier supérieur ou égal à 1, comprenant chacun au moins un catalyseur d'hydrotraitement, ladite section réactionnelle d'hydrotraitement étant alimentée au moins par ledit effluent hydrogéné issu de l'étape a) et un flux gazeux comprenant de l'hydrogène, ladite section réactionnelle d'hydrotraitement étant mise en oeuvre à une température entre 250 et 430°C, une pression partielle d'hydrogène entre 1,0 et 10,0 MPa abs. et une vitesse volumique horaire entre 0,1 et 10,0 h$^{-1}$, pour obtenir un effluent d'hydrotraitement ;
c) une étape d'hydrocraquage mise en oeuvre dans une section réactionnelle d'hydrocraquage, mettant en oeuvre au moins un réacteur à lit fixe ayant n lits catalytiques, n étant un nombre entier supérieur ou égal à 1, comprenant chacun au moins un catalyseur d'hydrocraquage, ladite section réactionnelle d'hydrocraquage étant alimentée au moins par ledit effluent hydrotraité issu de l'étape b) et un flux gazeux comprenant de l'hydrogène, ladite section réactionnelle d'hydrocraquage étant mise en oeuvre à une température entre 250 et 480°C, une pression partielle d'hydrogène entre 1,5 et 25,0 MPa abs. et une vitesse volumique horaire entre 0,1 et 10,0 h$^{-1}$, pour obtenir un effluent hydrocraqué ;
d) une étape de séparation, alimentée par l'effluent hydrocraqué issu de l'étape c) et une solution aqueuse, ladite étape étant opérée à une température entre 50 et 370°C, pour obtenir au moins un effluent gazeux, un effluent aqueux et un effluent hydrocarboné.

2. Procédé selon la revendication 1, lequel comprend en outre une étape e) fractionnement de tout ou partie de l'effluent hydrocarboné issu de l'étape d), pour obtenir au moins un flux gazeux et au moins deux flux hydrocarbonés liquides, lesdits deux flux hydrocarbonés liquides étant au moins une coupe naphta comprenant des composés ayant un point d'ébullition inférieur ou égal à 175°C et une coupe hydrocarbonée comprenant des composés ayant un point d'ébullition supérieur à 175°C.

3. Procédé selon la revendication 2, lequel comprend en outre une étape f) de recyclage dans laquelle au moins une fraction de la coupe comprenant des composés ayant un point d'ébullition supérieur à 175°C issue de l'étape e) de fractionnement est envoyée vers l'étape c) d'hydrocraquage.

4. Procédé selon l'une des revendications précédentes, lequel comprend en outre une étape g) de recyclage dans laquelle une fraction de l'effluent hydrocarboné issu de l'étape d) de séparation ou une fraction de la coupe naphta comprenant des composés ayant un point d'ébullition inférieur ou égal à 175°C issue de l'étape e) de fractionnement est envoyée vers l'étape a) d'hydrogénation sélective et/ou l'étape b) d'hydrotraitement.

5. Procédé selon l'une des revendications précédentes, lequel la quantité du flux de recycle des étapes f) et/ou g) est ajustée de sorte que le rapport pondéral entre le flux de recycle et la charge comprenant une huile de pyrolyse de plastiques est inférieur ou égal à 10.

6. Procédé selon l'une des revendications précédentes, comprenant une étape a0) de prétraitement de la charge comprenant une huile de pyrolyse de plastiques, ladite étape de prétraitement étant mise en oeuvre en amont de l'étape a) d'hydrogénation sélective et comprend une étape de filtration et/ou une étape d'un lavage à l'eau et/ou une étape d'adsorption.

7. Procédé selon l'une des revendications précédentes dans lequel la section réactionnelle de l'étape a) ou b) met en oeuvre aux moins deux réacteurs fonctionnant en mode permutable.

8. Procédé selon l'une des revendications précédentes dans lequel un flux contenant une amine est injectée en amont de l'étape a).

9. Procédé selon l'une des revendications précédentes dans lequel ledit catalyseur d'hydrogénation sélective comprend un support choisi parmi l'alumine, la silice, les silices-alumines, la magnésie, les argiles et leurs mélanges et une fonction hydro-déshydrogénante comprenant soit au moins un élément du groupe VIII et au moins un élément du groupe VIB, soit au moins un élément du groupe VIII.

10. Procédé selon l'une des revendications précédentes dans lequel ledit au moins un catalyseur d'hydrotraitement comprend un support choisi dans le groupe constitué par l'alumine, la silice, les silices-alumines, la magnésie, les argiles et leurs mélanges, et une fonction hydro-déshydrogénante comprenant au moins un élément du groupe VIII et/ou au moins un élément du groupe VIB.

11. Procédé selon l'une des revendications précédentes dans lequel ledit catalyseur d'hydrocraquage comprend un support choisi parmi les alumines halogénées, les combinaisons d'oxydes de bore et d'aluminium, les silice-alumines amorphes et les zéolithes et une fonction hydro-déshydrogénante comprenant au moins un métal du groupe VIB choisi parmi le chrome, le molybdène et le tungstène, seul ou en mélange, et/ou au moins un métal du groupe VIII choisi parmi le fer, le cobalt, le nickel, le ruthénium, le rhodium, le palladium et le platine.

12. Procédé selon la revendication précédente dans lequel ladite zéolithe est choisie parmi les zéolithes Y, seules ou en combinaison, avec d'autres zéolithes parmi les zéolithes beta, ZSM-12, IZM-2, ZSM-22, ZSM-23, SAPO-11, ZSM-48, ZBM-30, seules ou en mélange.

13. Procédé selon l'une des revendications précédentes, dans lequel l'effluent hydrocarboné issu de l'étape d) de séparation, ou au moins l'un des deux flux hydrocarboné(s) liquides issu(s) de l'étape e) optionnelle, est en tout ou partie envoyé vers une étape h) de vapocraquage réalisée dans au moins un four de pyrolyse à une température comprise entre 700 et 900°C et à une pression comprise entre 0,05 et 0,3 MPa relatif.

14. Procédé selon l'une des revendications 2 à 13, dans lequel la coupe naphta comprenant des composés ayant un point d'ébullition inférieur ou égal à 175°C issue de l'étape e) est fractionnée en une coupe naphta lourde comprenant des composés ayant un point d'ébullition entre 80 et 175°C et une coupe naphta légère comprenant des composés ayant un point d'ébullition inférieure à 80°C, au moins une partie de ladite coupe lourde étant envoyée vers un complexe aromatique comportant au moins une étape de reformage du naphta.

15. Procédé selon la revendication 14, dans lequel au moins une partie de la coupe naphta légère est envoyée dans l'étape h) de vapocraquage.

**Patentansprüche**

1. Verfahren zur Behandlung eines Einsatzmaterials, das ein Pyrolyseöl aus Kunststoffen umfasst, das Chlorverbindungen umfasst, umfassend:

   a) einen Schritt zur selektiven Hydrierung, der in einem Reaktionsabschnitt durchgeführt wird, dem zumindest das Einsatzmaterial und ein Gasstrom, der Wasserstoff umfasst, zugeführt wird, in Gegenwart von mindestens einem Katalysator für die selektive Hydrierung, bei einer Temperatur zwischen 100 und 280°C, einem Wasserstoffpartialdruck zwischen 1,0 und 10,0 MPa abs und einer Volumengeschwindigkeit pro Stunde zwischen 0,3 und 10,0 h$^{-1}$, um einen hydrierten Austrag zu erhalten;
   b) einen Hydrotreating-Schritt, der in einem Hydrotreating-Reaktionsabschnitt unter Verwendung von mindestens einem Festbettreaktor mit n Katalysatorbetten durchgeführt wird, wobei n eine Ganzzahl größer gleich 1 ist, das jeweils mindestens einen Hydrotreating-Katalysator umfasst, wobei dem Hydrotreating-Reaktionsabschnitt zumindest der hydrierte Austrag aus Schritt a) und ein Gasstrom, der Wasserstoff umfasst, zugeführt werden, wobei der Hydrotreating-Reaktionsabschnitt bei einer Temperatur zwischen 250 und 430°C, einem Wasserstoffpartialdruck zwischen 1,0 und 10,0 MPa abs und einer Volumengeschwindigkeit pro Stunde zwischen 0,1 und 10,0 h$^{-1}$ betrieben wird, um einen per Hydrotreating behandelten Austrag zu erhalten;
   b) einen Hydrocracking-Schritt, der in einem Hydrocracking-Reaktionsabschnitt unter Verwendung von mindestens einem Festbettreaktor mit n Katalysatorbetten durchgeführt wird, wobei n eine Ganzzahl größer gleich 1 ist, das jeweils mindestens einen Hydrocracking-Katalysator umfasst, wobei dem Hydrocracking-Reaktionsabschnitt zumindest der per Hydrotreating behandelte Austrag aus Schritt b) und ein Gasstrom, der Wasserstoff umfasst, zugeführt werden, wobei der Hydrocracking-Reaktionsabschnitt bei einer Temperatur zwischen 250 und 480°C, einem Wasserstoffpartialdruck zwischen 1,5 und 25,0 MPa abs und einer Volumengeschwindigkeit pro Stunde zwischen 0,1 und 10,0 h$^{-1}$ betrieben wird, um einen per Hydrocracking behandelten Austrag zu erhalten;
   d) einen Trennschritt, dem der per Hydrocracking behandelte Austrag aus Schritt c) und eine wässrige Lösung zugeführt werden, wobei der Schritt bei einer Temperatur zwischen 50 und 370°C durchgeführt wird, um mindestens einen Gasaustrag, einen wässrigen Austrag und einen Kohlenwasserstoffaustrag zu erhalten.

2. Verfahren nach Anspruch 1, das ferner einen Schritt e) zur Fraktionierung des gesamten oder eines Teils des Kohlenwasserstoffaustrags aus Schritt d) umfasst, um mindestens einen Gasstrom und mindestens zwei flüssige Kohlenwasserstoffströme zu erhalten, wobei die beiden flüssigen Kohlenwasserstoffströme mindestens eine Naphthafraktion, die Verbindungen mit einem Siedepunkt kleiner gleich 175°C umfasst, und eine Kohlenwasserstofffraktion sind, die Verbindungen mit einem Siedepunkt von über 175°C umfasst.

3. Verfahren nach Anspruch 2, das ferner einen Rückführschritt f) umfasst, wobei mindestens ein Anteil der Fraktion, die Verbindungen mit einem Siedepunkt von über 175°C umfasst, aus dem Fraktionierungsschritt e) zum Hydrocracking-Schritt c) geleitet wird.

4. Verfahren nach einem der vorhergehenden Ansprüche, das ferner einen Rückführschritt g) umfasst, wobei ein Anteil des Kohlenwasserstoffaustrags aus dem Trennschritt d) oder ein Anteil der Naphthafraktion, die Verbindungen mit einem Siedepunkt kleiner gleich 175°C umfasst, aus dem Fraktionierungsschritt e) zum Schritt a) zur selektiven Hydrierung und/oder zum Hydrotreating-Schritt b) geleitet wird.

5. Verfahren nach einem der vorhergehenden Ansprüche, wobei die Menge des Rückführstroms aus Schritt f) und/oder g) derart angepasst wird, dass das Gewichtsverhältnis zwischen dem Rückführstrom und dem Einsatzmaterial, das ein Pyrolyseöl aus Kunststoffen umfasst, kleiner gleich 10 beträgt.

6. Verfahren nach einem der vorhergehenden Ansprüche, das einen Schritt a0) zur Vorbehandlung des Einsatzmaterials umfasst, das ein Pyrolyseöl aus Kunststoffen umfasst, wobei der Schritt zur Vorbehandlung vor Schritt a) zur selektiven Hydrierung durchgeführt wird und einen Filterschritt und/oder einen Schritt zum Waschen mit Wasser und/oder einen Adsorptionsschritt umfasst.

7. Verfahren nach einem der vorhergehenden Ansprüche, wobei in dem Reaktionsabschnitt von Schritt a) oder b) mindestens zwei Reaktoren eingesetzt werden, die im Wechselbetrieb arbeiten.

8. Verfahren nach einem der vorhergehenden Ansprüche, wobei vor Schritt a) ein Strom eingeleitet wird, der ein Amin enthält.

9. Verfahren nach einem der vorhergehenden Ansprüche, wobei der Katalysator zur selektiven Hydrierung einen Träger, der ausgewählt ist aus Aluminiumoxid, Siliciumoxid, Siliciumoxid-Aluminiumoxiden, Magnesiumoxid, Tonen und ihren Mischungen, sowie eine hydrierend-dehydrierende Funktion umfasst, die entweder ein Element der Gruppe VIII und mindestens ein Element der Gruppe VI B oder mindestens ein Element der Gruppe VIII umfasst.

10. Verfahren nach einem der vorhergehenden Ansprüche, wobei der Hydrotreating-Katalysator einen Träger, der ausgewählt ist aus der Gruppe, die aus Aluminiumoxid, Siliciumoxid, Siliciumoxid-Aluminiumoxiden, Magnesiumoxid, Tonen und ihren Mischungen besteht, sowie eine hydrierend-dehydrierende Funktion umfasst, die mindestens ein Element der Gruppe VIII und/oder mindestens ein Element der Gruppe VI B umfasst.

11. Verfahren nach einem der vorhergehenden Ansprüche, wobei der Hydrocracking-Katalysator einen Träger, der ausgewählt ist aus halogenierten Aluminiumoxiden, Kombinationen aus Bor- und Aluminiumoxiden, amorphen Siliciumoxid-Aluminiumoxiden und Zeolithen, sowie eine hydrierend-dehydrierende Funktion umfasst, die mindestens ein Metall der Gruppe VI B, das aus Chrom, Molybdän und Wolfram, allein oder gemischt, ausgewählt ist, und/oder mindestens ein Metall der Gruppe VIII umfasst, das aus Eisen, Cobalt, Nickel, Ruthenium, Rhodium, Palladium und Platin ausgewählt ist.

12. Verfahren nach dem vorhergehenden Anspruch, wobei das Zeolith aus Y-Zeolithen Y, allein oder kombiniert mit anderen Zeolithen unter Beta-Zeolithen, ZSM-12, IZM-2, ZSM-22, ZSM-23, SAPO-11, ZSM-48, ZBM-30, allein oder gemischt, ausgewählt ist.

13. Verfahren nach einem der vorhergehenden Ansprüche, wobei der Kohlenwasserstoffaustrag aus dem Trennschritt d) oder mindestens einer der beiden flüssigen Kohlenwasserstoffströme aus dem optionalen Schritt e) vollständig oder teilweise zu einem Steamcracking-Schritt h) geleitet wird, der in mindestens einem Pyrolyseofen bei einer Temperatur zwischen 700 und 900°C und bei einem Relativdruck zwischen 0,05 und 0,3 MPa durchgeführt wird.

14. Verfahren nach einem der Ansprüche 2 bis 13, wobei die Naphthafraktion, die Verbindungen mit einem Siedepunkt kleiner gleich 175°C umfasst, aus Schritt e) in eine schwere Naphthafraktion, die Verbindungen mit einem Siedepunkt zwischen 80°C und 175 °C umfasst, und eine leichte Naphthafraktion fraktioniert wird, die Verbindungen mit einem Siedepunkt unter 80°C umfasst, wobei mindestens ein Teil der schweren Naphthafraktion zu einem Aromatenkomplex geleitet wird, der mindestens einen Schritt zum Reformieren von Naphtha aufweist.

15. Verfahren nach Anspruch 14, wobei mindestens ein Teil der leichten Naphthafraktion zum Steamcracking-Schritt h) geleitet wird.

**Claims**

1. Process for treating a feedstock comprising a plastics pyrolysis oil comprising chlorinated compounds, comprising:

   a) a selective hydrogenation step performed in a reaction section fed at least with said feedstock and a gas stream comprising hydrogen, in the presence of at least one selective hydrogenation catalyst, at a temperature of between 100 and 280°C, a hydrogen partial pressure of between 1.0 and 10.0 MPa abs. and an hourly space velocity of between 0.3 and 10.0 h$^{-1}$, to obtain a hydrogenated outflow;
   b) a hydrotreatment step performed in a hydrotreatment reaction section, using at least one fixed-bed reactor having n catalytic beds, n being an integer greater than or equal to 1, each comprising at least one hydrotreatment catalyst, said hydrotreatment reaction section being fed at least with said hydrogenated outflow from step a) and a gas stream comprising hydrogen, said hydrotreatment reaction section being operated at a temperature of between 250 and 430°C, a hydrogen partial pressure of between 1.0 and 10.0 MPa abs. and an hourly space velocity of between 0.1 and 10.0 $h^{-1}$, to obtain a hydrotreatment outflow;

c) a hydrocracking step performed in a hydrocracking reaction section, using at least one fixed-bed reactor having n catalytic beds, n being an integer greater than or equal to 1, each comprising at least one hydrocracking catalyst, said hydrocracking reaction section being fed at least with said hydrotreated outflow from step b) and a gas stream comprising hydrogen, said hydrocracking reaction section being operated at a temperature of between 250 and 480°C, a hydrogen partial pressure of between 1.5 and 25.0 MPa abs. and an hourly space velocity of between 0.1 and 10.0 $h^{-1}$, to obtain a hydrocracked outflow;

d) a separation step, fed with the hydrocracked outflow from step c) and an aqueous solution, said step being performed at a temperature of between 50 and 370°C, to obtain at least one gaseous outflow, an aqueous outflow and a hydrocarbon-containing outflow.

2. Process according to Claim 1, which also comprises a step e) of fractionating all or a portion of the hydrocarbon-containing outflow from step d), to obtain at least one gas stream and at least two liquid hydrocarbon-containing streams, said two liquid hydrocarbon-containing streams being at least one naphtha cut comprising compounds having a boiling point of less than or equal to 175°C and a hydrocarbon-containing cut comprising compounds having a boiling point of greater than 175°C.

3. Process according to Claim 2, which also comprises a recycling step f) wherein at least a fraction of the cut comprising compounds having a boiling point of greater than 175°C from the fractionation step e) is sent to the hydrocracking step c).

4. Process according to one of the preceding claims, which also comprises a recycling step g) wherein a fraction of the hydrocarbon-containing outflow from the separation step d) or a fraction of the naphtha cut comprising compounds having a boiling point of less than or equal to 175°C from the fractionation step e) is sent to the selective hydrogenation step a) and/or the hydrotreatment step b).

5. Process according to one of the preceding claims, wherein the amount of the recycle stream from steps f) and/or g) is adjusted so that the weight ratio between the recycle stream and the feedstock comprising a plastics pyrolysis oil is less than or equal to 10.

6. Process according to one of the preceding claims, comprising a step a0) of pretreating the feedstock comprising a plastics pyrolysis oil, said pretreatment step being performed upstream of the selective hydrogenation step a) and comprises a filtration step and/or a step of washing with water and/or an adsorption step.

7. Process according to one of the preceding claims, wherein the reaction section in step a) or b) employs at least two reactors operating in an interchangeable manner.

8. Process according to one of the preceding claims, wherein a stream containing an amine is injected upstream of step a).

9. Process according to one of the preceding claims, wherein said selective hydrogenation catalyst comprises a support selected from alumina, silica, silica-aluminas, magnesia, clays and mixtures thereof and a hydrogenating-dehydrogenating function comprising either at least one group VIII element and at least one group VIB element, or at least one group VIII element.

10. Process according to one of the preceding claims, wherein said at least one hydrotreatment catalyst comprises a support selected from the group consisting of alumina, silica, silica-aluminas, magnesia, clays and mixtures thereof and a hydrogenating-dehydrogenating function comprising at least one group VIII element and/or at least one group VIB element.

11. Process according to one of the preceding claims, wherein said hydrocracking catalyst comprises a support selected from halogenated aluminas, combinations of oxides of boron and aluminium, amorphous silica-aluminas, and zeolites and a hydrogenating-dehydrogenating function comprising at least one group VIB metal selected from chromium, molybdenum and tungsten, alone or as a mixture, and/or at least one group VIII metal selected from iron, cobalt, nickel, ruthenium, rhodium, palladium and platinum.

12. Process according to the preceding claim, wherein said zeolite is selected from Y zeolites, alone or in combination, with other zeolites from among beta, ZSM-12, IZM-2, ZSM-22, ZSM-23, SAPO-11, ZSM-48 and ZBM-30 zeolites, alone or as a mixture.

13. Process according to one of the preceding claims, wherein the hydrocarbon-containing outflow from the separation step d), or at least one of the two liquid hydrocarbon-containing streams from the optional step e), is sent in its entirety or in part to a steam cracking step h) performed in at least one pyrolysis furnace at a temperature of between 700 and 900°C and at a pressure of between 0.05 and 0.3 MPa relative.

14. Process according to one of Claims 2 to 13, wherein the naphtha cut comprising compounds having a boiling point of less than or equal to 175°C from step e) is fractionated into a heavy naphtha cut comprising compounds having a boiling point of between 80 and 175°C and a light naphtha cut comprising compounds having a boiling point of less than 80°C, at least a portion of said heavy cut being sent to an aromatic complex including at least one naphtha reforming step.

15. Process according to Claim 14, wherein at least a portion of the light naphtha cut is sent to the steam cracking step h).

**Figure 1**

**Figure 2**

**Figure 3**

**Figure 4**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2018055555 A
- FR 2001758
- WO 2014001632 A
- US 3492220 A
- US 5969201 A
- FR 2681871
- FR 3051375
- EP 0113297 A
- EP 0113284 A
- US 5221656 A
- US 5827421 A
- US 7119045 B
- US 5622616 A
- US 5089463 A
- US 6589908 B
- US 4818743 A
- US 6332976 B
- CN 102051202
- US 2007080099 A

**Littérature non-brevet citée dans la description**

- **D.R. LIDE.** CRC Handbook of Chemistry and Physics. CRC press, 2000
- *The Journal of the American Chemical Society,* 1938, vol. 6Q, 309
- **C. LÓPEZ-GARCÍA et al.** Near Infrared Monitoring of Low Conjugated Diolefins Content in Hydrotreated FCC Gasoline Streams. *Oil & Gas Science and Technology - Rev. IFP,* 2007, vol. 62 (1), 57-68